# FASCICULE DE BREVET EUROPEEN

(11) **EP 3 853 655 B1**
(45) Date de publication et mention de la délivrance du brevet: **31.08.2022**
(21) Numéro de dépôt: 19779371.4
(22) Date de dépôt: 10.09.2019
(51) Int. Cl.: G02B 27/00, A61B 1/00, G02B 23/24

(54) **DISPOSITIFS ET MÉTHODES DE TRANSPORT ET DE CONTRÔLE DE FAISCEAUX LUMINEUX**
VORRICHTUNGEN UND VERFAHREN ZUM TRANSPORTIEREN UND STEUERN VON LICHTSTRAHLEN
DEVICES AND METHODS FOR TRANSPORTING AND CONTROLLING LIGHT BEAMS

(30) Priorité: 20.09.2018 FR 1858545
(43) Date de publication de la demande: 28.07.2021
(73) Titulaire: Centre national de la recherche scientifique, 75016 Paris (FR); Université d'Aix-Marseille, 13007 Marseille (FR); Université de Lille, 59000 Lille (FR); Ecole Centrale De Marseille (ECM), 13013 Marseille (FR)
(72) Inventeur: RIGNEAULT, Hervé, 13190 Allauch (FR); BOUWMANS, Géraud, 59830 Cysoing (FR); ANDRESEN, Esben, 59800 Lille (FR); SIVANKUTTY, Siddharth, 13001 Marseille (FR); TSVIRKUN, Viktor, 13008 Marseille (FR); VANVINCQ, Olivier, 59240 Dunkerque (FR)
(74) Mandataire: Osha Liang
(86) Numéro de dépôt international: PCT/EP2019/074142
(87) Numéro de publication internationale: WO 2020/058043

(56) Documents cités:
- EP-A1- 2 743 743
- FR-A1- 3 049 719
- US-A1- 2014 276 111
- V. TSVIRKUN ET AL.: "Bending-induced inter-core group delays in multicore fibers", OPTICS EXPRESS, vol. 25, 2017, pages 31863-31875, XP002791610, cité dans la demande

## Description

### Domaine technique de l'invention

La présente invention concerne des dispositifs et méthodes de transport et de contrôle de faisceaux lumineux, notamment pour l'imagerie endo-microscopique dite « sans lentille ». Elle s'applique par exemple à l'exploration endoscopique des organes chez un être vivant, humain ou animal.

### Etat de l'art

Les développements en imagerie endo-microscopique nécessitent l'utilisation de dispositifs optomécaniques fibrés présentant des spécificités par rapport aux systèmes imageurs en espace libre.

En effet, la construction d'un microscope miniature qui comprendrait une source lumineuse, une optique de focalisation et une caméra à l'extrémité distale (c'est-à-dire situé en bout de fibre, du côté de l'échantillon) d'un endoscope médical n'est pas envisageable du fait de l'encombrement de l'ensemble des composants. On cherche de ce fait des solutions permettant de réaliser une imagerie en bout de fibre optique tout en limitant l'encombrement à l'extrémité distale de l'endoscope.

Il est connu notamment une technologie qualifiée d'« endoscopie sans lentille », et décrite par exemple dans Cizmar et al. « Exploiting multimode waveguides for pure fibre-based imaging », Nat. Commun. 3, 1027 (2012). Cette technique est basée sur l'utilisation d'une fibre optique multimodes, ou MMF selon l'abréviation de l'expression anglo-saxonne « Multi-Mode Fiber ». La fibre optique MMF est éclairée avec une source cohérente. Du côté proximal (c'est-à-dire en entrée de fibre optique, du côté opposé à l'échantillon) de la fibre optique MMF, un modulateur spatial de front d'onde (SLM pour « Spatial Light Modulator ») permet de jouer sur les modes de propagation de la fibre de telle sorte que l'addition cohérente de ces modes permet de générer la figure d'intensité recherchée à l'extrémité distale de la fibre MMF. Dans un mode de réalisation, on cherche par exemple à produire un point focal à l'extrémité distale de la fibre MMF et à balayer l'échantillon pour obtenir une image comme on le ferait dans un montage classique de microscopie confocale.

Cette technique, extrêmement puissante du fait du caractère déterministe de la matrice de transmission de la fibre qui relie un champ entrant en partie proximale de la fibre avec un champ sortant en partie distale (et vice versa), permet de s'affranchir de toute optique du côté distal de la fibre multimodes et de ce fait de réduire l'encombrement.

Cependant, la matrice de transmission de la fibre est fortement dépendante de la courbure de la fibre optique MMF. L'imagerie endo-microscopique au moyen d'une fibre optique MMF est donc extrêmement sensible à tout mouvement de la fibre. Par ailleurs, du fait du caractère multimode, une impulsion courte en partie proximale est allongée en partie distale, ce qui limite les possibilités d'application à l'imagerie non linéaire qui nécessite de travailler avec des impulsions lumineuses de fortes intensités crêtes.

En parallèle des technologies basées sur l'utilisation de fibres multimodes, une technologie également de type « sans lentille » s'est développée, basée sur l'utilisation d'un paquet de fibres optiques monomodes (voir par exemple French et al. brevet US 8,585,587). Selon la technique décrite, un modulateur spatial de front d'onde (SLM) agencé du côté proximal du paquet de fibres optiques monomodes permet de contrôler à l'extrémité distale du paquet de fibres le front d'onde émis par une source lumineuse. Le caractère monomode des fibres élimine toute dispersion intermodale ; ainsi, la seule contribution à la dispersion est la dispersion chromatique qui est la même pour toutes les fibres optiques monomodes et qui peut donc être compensée de manière globale. De ce fait, l'utilisation d'un paquet de fibres optiques monomodes est préférée par rapport aux fibres multimodes pour la propagation d'impulsions courtes.

Diverses publications ont décrit des variantes d'endo-microscopie sans lentille basées sur l'utilisation d'un paquet de fibres optiques monomodes et plus précisément d'une fibre multicoeur ou « MCF » selon l'expression anglo-saxonne « Multi-Core Fiber ». Ainsi par exemple, il est montré comment on peut accéder, en partie distale du paquet de fibres optiques, à un balayage très rapide du point de focalisation, en imprimant au moyen d'un dispositif galvanométrique un angle variable du front d'onde en entrée du SLM (voir par exemple E.R. Andresen et al. « Toward endoscopes with no distal optics: video-rate scanning microscopy through a fiber bundle », Opt. Lett. Vol. 38, N°5, 609-611 (2013)). Dans E.R. Andresen et al. (« Two-photon lensless endoscope», Opt. Express 21, N°18 , 20713-20721 (2013)), les auteurs ont démontré la faisabilité expérimentale d'un système d'imagerie non linéaire bi-photonique (TPEF) en endo-microscopie sans lentille. Dans E.R. Andresen et al. (« Measurement and compensation of residual group delay in a multi-core fiber for lensless endoscopy", JOSA B, Vol. 32, No. 6, 1221 - 1228 (2015)), il est décrit un dispositif de contrôle des retards de vitesse de groupe (ou « GDC » pour « Group Delay Control ») pour le transport et le contrôle d'impulsions lumineuses dans un système d'imagerie endo-microscopique sans lentille basé sur l'utilisation d'un paquet de fibres optiques monomodes.

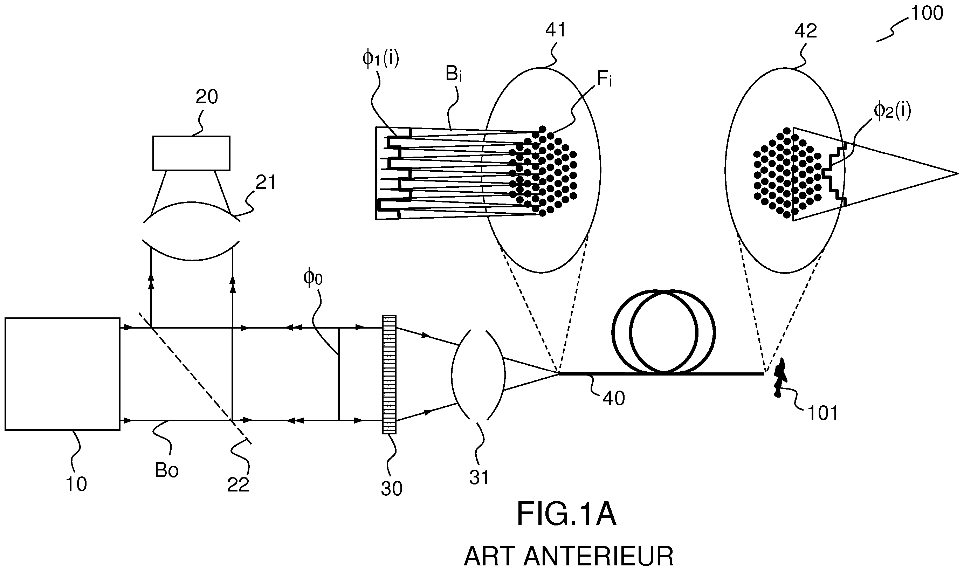

La FIG. 1A illustre de façon schématique un système d'imagerie endo-microscopique sans lentille 100 utilisant un paquet de fibres optiques monomodes. Le système d'imagerie 100 comprend généralement une source d'émission 10 pour l'émission d'un faisceau lumineux incident Bo, continu ou formé d'impulsions dans le cas de l'application à l'imagerie non linéaire. Le système 100 comprend par ailleurs une voie de détection comprenant un objectif 21 et un détecteur 20. La voie de détection est séparée de la voie d'émission par une lame séparatrice 22. Le système d'imagerie 100 comprend également un dispositif de transport et de contrôle des faisceaux lumineux permettant d'éclairer un objet d'analyse 101 déporté. Le dispositif de transport et de contrôle comprend un paquet de fibres optiques monomodes 40 avec une face d'entrée (ou face proximale) 41 et une face de sortie (ou face distale) 42 représentées de façon agrandie sur la figure 1A, et un modulateur spatial de front d'onde (« SLM ») 30 agencé à l'extrémité proximale du paquet de fibres 40 et permettant de contrôler le front d'onde du faisceau émis par la source 10. Le modulateur spatial de lumière permet d'imprimer sur le front d'onde entrant, présentant une fonction de phase Φ₀, un déphasage donné Φ₁(i) pour chaque faisceau élémentaire Bᵢ destiné à entrer dans une fibre optique Fᵢ du paquet de fibres 40. La fonction de phase Φ₁(i) pourra être telle que, par exemple, après propagation dans le paquet de fibres optiques, l'onde présente une phase parabolique Φ₂(i) en champ lointain. Cette phase parabolique permet au faisceau de se focaliser du côté distal sur l'objet d'analyse 101 alors qu'il n'y a aucune lentille physique présente ; c'est l'origine de la terminologie « endoscope sans lentille ». Plus généralement, on pourra induire une fonction de phase Φ₁(i) adaptée à générer, en sortie du paquet de fibres optiques, une figure d'intensité donnée. Par ailleurs, il est possible grâce au modulateur spatial de lumière de compenser des déphasages intrinsèques introduits par chacune des fibres optiques Fᵢ.

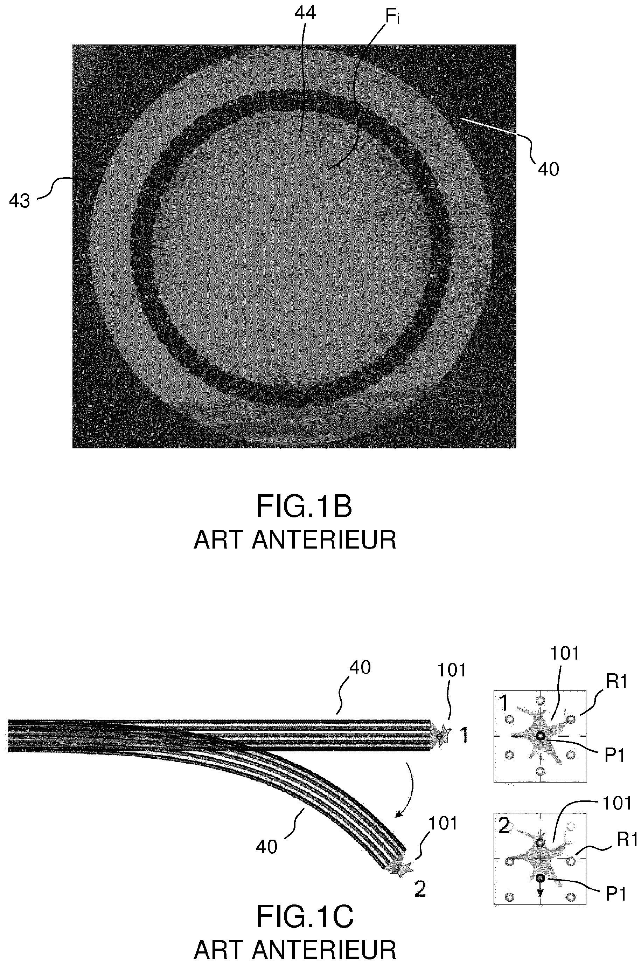

Le paquet de N fibres optiques monomodes peut être formé d'un ensemble de fibres optiques monomodes individuelles, comportant chacune un coeur et une gaine, typiquement une centaine à quelques dizaines de milliers de fibres, rassemblées sous forme d'un faisceau de fibres ; le paquet de N fibres optiques monomodes peut également être formé d'un ensemble de coeurs monomodes d'une fibre multicoeur, de préférence au moins une centaine de coeurs monomodes, comme cela est illustré sur la figure 1B. Ainsi, dans l'exemple de la FIG. 1B, une fibre multicœur 40 est représentée qui comprend un ensemble de coeurs monomodes Fᵢ, une gaine externe 43 et également dans cet exemple une gaine interne multimodes 44 adaptée pour collecter le signal lumineux rétrodiffusé par l'objet d'analyse depuis l'extrémité distale jusqu'à l'extrémité proximale.

Un avantage de la technique « sans lentille » ainsi décrite est notamment de présenter une plus faible sensibilité aux mouvements de la fibre que d'autres techniques, et notamment les fibres optiques MMF précédemment décrites. En effet, les modes des fibres optiques monomodes sont localisés et confinés en des régions définies sur la surface transverse du paquet de fibres optiques.

Cependant, comme cela est illustré sur la FIG. 1C, une courbure du paquet de fibres optiques résulte en une translation de la figure d'intensité en sortie du paquet de fibres. En effet, la courbure du paquet de fibres entraîne un allongement du chemin optique pour les coeurs situés à l'extérieur de la courbure et au contraire, une diminution du chemin optique pour les coeurs situés à intérieur de la courbure. Il en résulte l'introduction d'un déphasage additionnel entre les coeurs du paquet de fibres monomodes. Ce déphasage additionnel, fonction de la distance d'un coeur (i) au coeur central, entraîne un déplacement en sortie du paquet de fibres de la fonction d'étalement du point du système d'imagerie (ou « PSF » selon l'expression anglo-saxonne « *Point Spread Function* »), la PSF étant la réponse spatiale impulsionnelle du système d'imagerie défini par le SLM et le paquet de fibres optiques monomodes. Ainsi par exemple, comme cela est illustré sur la FIG. 1C, la figure d'intensité ou PSF représentée dans cet exemple par un point de focalisation P₁ et des répliques R₁ se trouve translatée par rapport à l'objet d'observation 101 entre la situation (1) dans laquelle le paquet de fibres ne subit pas de courbure et la situation (2) dans laquelle le paquet de fibres subit une courbure. Ce déplacement de la PSF est gênant car lors d'une observation *in vivo* par exemple d'un organe, il en résulte un changement de la zone observée de l'objet en cas de modification de la courbure du paquet de fibres optiques monomodes.

La présente invention propose des dispositifs et méthodes de transport et de contrôle de faisceaux lumineux, notamment pour systèmes d'imagerie endo-microscopiques dit « sans lentille », qui rendent invariante spatialement la PSF en cas de courbure du paquet de fibres optiques, c'est-à-dire qui permettent le maintien de la figure d'intensité dans le repère de l'extrémité distale du paquet de fibres.

### RESUME DE L'INVENTION

Selon un premier aspect, la présente description concerne un dispositif de transport et de contrôle de faisceaux lumineux comprenant :
- un guide de lumière comprenant un paquet de fibres optiques monomodes non couplées, chaque fibre optique monomode étant destinée à recevoir un faisceau lumineux élémentaire à une extrémité proximale et à émettre un faisceau lumineux à une extrémité distale, ledit paquet de fibres optiques monomodes comprenant, en opération, un rayon de courbure minimal correspondant à une courbure maximale du paquet de fibres;
- un dispositif optique de contrôle de la phase agencé du côté de l'extrémité proximale du guide de lumière comprenant :
   - au moins un premier modulateur spatial de lumière adapté pour l'application d'un déphasage sur chacun des faisceaux élémentaires ;
   - une unité de contrôle du premier modulateur spatial de lumière configurée pour appliquer un déphasage sur chacun des faisceaux élémentaires pour former à l'extrémité distale du guide de lumière un faisceau d'illumination avec une fonction de phase prédéterminée,
et dans lequel ledit paquet de fibres optiques monomodes est torsadé, et comprend une période de torsade déterminée pour conserver ladite fonction de phase à l'extrémité distale du guide de lumière lorsque le paquet de fibres optiques monomodes subit une courbure inférieure à ladite courbure maximale.

La fonction de phase est déterminée de telle sorte que la PSF, également appelée figure d'intensité dans la présente description, comprenne par exemple un ou plusieurs points de focalisation ou une figure d'intensité prédéfinie en fonction de la forme de l'objet à imager, ou une figure de speckle, c'est-à-dire une fonction de phase aléatoire en sortie.

Par conservation de la fonction de phase, on entend le maintien du déphasage entre les fibres optiques monomodes à moins de 2π lorsque le paquet de fibres optiques torsadé subit une courbure inférieure à ladite courbure maximale.

Le maintien du déphasage peut se mesurer en injectant de la lumière dans deux fibres optiques monomodes du paquet de fibre ; la figure d'intensité au bout du paquet de fibres est alors constituée de franges ; lorsque le paquet de fibres subit une courbure, un déphasage inférieur à 2π correspond à un maintien de la figure d'intensité à une frange près.

Les déposants ont montré que dans un dispositif de transport et de contrôle de faisceaux lumineux ainsi défini, la fonction d'intensité à l'extrémité distale du guide de lumière est très peu perturbée, en opération, lors de courbures subies par le paquet de fibres optiques. Il en résulte la possibilité d'une imagerie dynamique où l'on peut continuer à imager un objet même lorsque le paquet de fibres est courbé.

Au sens de la présente description, des fibres monomodes non couplées sont des fibres présentant un couplage inférieur à -20dB/m, avantageusement inférieur à -30dB/m. Des fibres non couplées permettent le transport et le contrôle des faisceaux optiques sur une grande longueur du paquet de fibres, tout en offrant la possibilité de compenser les déphasages inter-cœurs.

Selon un ou plusieurs exemples de réalisation, la courbure maximale pourra ne pas dépasser la courbure que peut subir une fibre optique monomode en maintenant des pertes optiques inférieures à une valeur de seuil donnée, par exemple 1 dB/m (correspondant à une transmission de 80% de l'énergie au bout de 1 m de fibres), avantageusement inférieures à 0,5 dB/m. Typiquement, cela correspond à un rayon minimal de courbure supérieur ou égal à environ 2,5 mm.

En pratique cependant, une courbure maximale du paquet de fibres optiques monomodes pourra être définie en fonction de l'application. En effet, en fonction de l'application, par exemple en endo-microscopie, le paquet de fibres optiques monomodes pourra être plus ou moins courbé selon les objets à imager.

Ainsi, selon un ou plusieurs exemples de réalisation, la courbure maximale, en opération, d'un paquet de fibres optiques monomodes pourra ainsi correspondre à un rayon minimal de courbure supérieur ou égal à 5 cm, ou supérieur ou égal à 10 cm, ou supérieur ou égal à 20 cm.

Selon un ou plusieurs exemples de réalisation, la longueur du paquet de fibres optiques monomodes est égale à *k* fois la période de torsade où *k* est un entier. Les déposants ont montré que cette configuration est optimale pour avoir la meilleure conservation de la fonction de phase à l'extrémité distale du paquet de fibres, quelle que soit la courbure maximale du paquet de fibres optiques monomodes.

Cependant, si cette condition n'est pas respectée, les déposants ont montré que pour un demi-diamètre donné du paquet de fibres optiques monomodes (distance du coeur central à un coeur périphérique le plus éloigné), on peut définir une gamme de périodes de torsade en fonction de la courbure maximale subie par le paquet de fibres optiques en opération, afin de conserver la fonction de phase à l'extrémité distale du guide de lumière.

Selon un ou plusieurs exemples de réalisation, le demi-diamètre du paquet de fibres optiques monomodes est compris entre 100 et 500 µm, avantageusement entre 100 et 300 µm, avantageusement entre 100 et 200 µm.

Selon un ou plusieurs exemples de réalisation, la période de torsade est supérieure à 1 mm, avantageusement supérieure à 2,5 mm, pour limiter les pertes optiques résultant de la torsade.

En pratique, pour des applications par exemple en endo-microscopie, on pourra choisir une période de torsade comprise entre 1 mm et 30 mm, avantageusement entre 2,5 mm et 10 mm.

Selon un ou plusieurs exemples de réalisation, l'unité de contrôle du premier modulateur spatial de lumière est configurée en outre pour appliquer une déviation angulaire sur chacun des faisceaux élémentaires à l'entrée proximale dudit paquet de fibres optiques monomodes torsadé, ladite déviation angulaire étant déterminée en fonction de la position de la fibre monomode destinée à recevoir ledit faisceau élémentaire dans ledit paquet de fibres optiques monomodes pour assurer un couplage optimal (ou injection de lumière) dans ladite fibre monomode. Les déposants ont montré que cette correction angulaire du côté proximal du paquet de fibres optiques monomodes permettait de conserver une très bonne transmission de la lumière, y compris dans les fibres monomodes périphériques les plus éloignées de la fibre centrale.

Selon un ou plusieurs exemples de réalisation, le guide de lumière comprend à l'extrémité distale et/ou à l'extrémité proximale dudit paquet de fibres optiques monomodes torsadé un tronçon de période de torsade variable, dans lequel la période de torsade tend vers l'infini du côté de ladite extrémité distale et/ou proximale. Autrement dit, à l'interface du tronçon avec l'espace libre, toutes les fibres optiques monomodes sont parallèles. Cette configuration permet également d'optimiser le couplage dans le paquet de fibres torsadé. Aucune correction angulaire n'est alors requise du côté proximal.

Selon un ou plusieurs exemples de réalisation, le ou les tronçon(s) de période de torsade variable présentent une longueur inférieure à 5 cm.

Selon un ou plusieurs exemples de réalisation, ledit tronçon de torsade variable présente à ladite extrémité distale et/ou proximale un tronçon non torsadé présentant une longueur inférieure à 1 cm.

Selon un ou plusieurs exemples de réalisation, le dispositif selon le premier aspect est adapté au transport et au contrôle de faisceaux lumineux comprenant des impulsions optiques, par exemple des impulsions de durées d'impulsions comprises entre 100 fs et 10 ns. Ledit dispositif peut alors comprendre en outre un dispositif de contrôle des retards de vitesse de groupe des impulsions lumineuses configuré pour supprimer les retards statiques de vitesse de groupe entre les fibres monomodes dudit paquet de fibres optiques monomodes torsadé.

Selon un ou plusieurs exemples de réalisation, au moins une partie desdites fibres optiques monomodes sont dopées.

Selon un ou plusieurs exemples de réalisation, le guide de lumière comprend un paquet de N fibres optiques monomodes formé d'un ensemble de N fibres optiques monomodes individuelles, comprenant chacune un coeur et une gaine, typiquement une centaine à quelques dizaines de milliers de fibres, rassemblées sous forme d'un faisceau de fibres.

Selon un ou plusieurs exemples de réalisation, le guide de lumière comprend un paquet de N fibres optiques monomodes formé d'un ensemble de coeurs monomodes, de préférence au moins une centaine. Par exemple, le guide de lumière est une fibre multicoeur et le paquet de N fibres optiques monomodes est formé par les coeurs monomodes de la fibre multicœurs.

Selon un ou plusieurs exemples de réalisation, le guide de lumière comprend une fibre multicoeurs à double gaine ; une telle fibre multicoeurs présente l'avantage de transporter avec une grande efficacité le signal lumineux rétrodiffusé dans une gaine de la fibre multicoeurs double gaine, généralement une gaine multimode.

Par fibre optique monomode, on comprend une fibre dans laquelle la lumière ne peut se propager que dans un seul mode du champ électromagnétique ; par extension on comprend aussi une fibre dite 'monomode effective' qui comprend plusieurs modes mais dans laquelle les conditions de couplage n'excitent qu'un seul mode (généralement le mode fondamental) qui confine la lumière durant toute la propagation (pas de couplage ou couplage très faible avec les autres modes).

Dans l'ensemble de la description, on pourra utiliser le terme « coeur monomode » ou « une fibre optique monomode » pour évoquer indifféremment aussi bien une fibre optique monomode individuelle qu'un coeur monomode d'une fibre multicœurs.

Selon un ou plusieurs exemples de réalisation, le premier modulateur spatial de lumière comprend un miroir déformable segmenté ou à membrane, pour un fonctionnement en réflexion.

Selon un ou plusieurs exemples de réalisation, le premier modulateur spatial de lumière comprend une matrice de cristaux liquides, pour un fonctionnement en réflexion ou en transmission.

Selon un ou plusieurs exemples de réalisation, l'unité de contrôle du premier modulateur spatial de lumière est configurée pour appliquer un déphasage sur chacun des faisceaux élémentaires pour former à l'extrémité distale du guide de lumière un faisceau d'illumination avec une fonction de phase évolutive en fonction du temps. Il sera par exemple ainsi possible de faire un balayage de la zone à illuminer.

Selon un ou plusieurs exemples de réalisation, les fibres monomodes sont agencées de façon périodique ou quasi-périodique au sein du paquet de fibres.

Selon un ou plusieurs exemples de réalisation, les fibres monomodes sont agencées de façon apériodique au sein du paquet de fibres, ce qui permet de réduire notablement l'intensité de « répliques » dans le plan image, c'est-à-dire des points de focalisation de plus faible intensité qui entourent un point de focalisation central plus lumineux.

Selon un deuxième aspect, la présente description concerne un système d'imagerie endo-microscopique comprenant une source lumineuse; un dispositif selon le premier aspect pour le transport et le contrôle des faisceaux lumineux émis par ladite source afin de former un faisceau d'illumination d'un objet avec une fonction de phase déterminée; et une voie de détection destinée à la détection de la lumière renvoyée par l'objet et transmise à travers ledit au moins un guide de lumière, de son extrémité distale à son extrémité proximale.

Selon un troisième aspect, la présente description concerne un procédé de transport et de contrôle de faisceaux lumineux comprenant :
- la réception de faisceaux lumineux élémentaires à une extrémité proximale d'un paquet de N fibres optiques monomodes d'un guide de lumière, dans lequel :
   o chaque fibre optique monomode est destinée à recevoir un faisceau lumineux élémentaire et à émettre un faisceau lumineux à une extrémité distale ;
   o ledit paquet de fibres optiques monomodes comprend, en opération, un rayon de courbure minimal correspondant à une courbure maximale du paquet de fibres; et
   ∘ ledit paquet de fibres optiques monomodes est torsadé, et comprend une période de torsade ;

- l'application au moyen d'au moins un premier modulateur spatial de lumière agencé du côté de l'extrémité proximale dudit paquet de fibres optiques monomodes d'un déphasage sur chacun des faisceaux élémentaires, afin de former à l'extrémité distale du guide de lumière un faisceau d'illumination avec une fonction de phase déterminée, ladite période de torsade étant déterminée pour conserver ladite fonction de phase à l'extrémité distale du guide de lumière lorsque le paquet de fibres optiques monomodes subit une courbure inférieure à ladite courbure maximale.

Selon un ou plusieurs exemples de réalisation, l'application du déphasage sur chacun des faisceaux élémentaires vise à inscrire à l'extrémité distale du tronçon de fibre optique multimodes une fonction de phase déterminée pour former un faisceau d'illumination convergent à une distance donnée d'une face de sortie du tronçon de fibre optique multimodes, permettant de former un ou plusieurs point(s) de focalisation.

Selon un ou plusieurs exemples de réalisation, l'application de déphasages successifs sur chacun des faisceaux élémentaires permet un balayage du point de focalisation dans un plan à ladite distance donnée de la face de sortie du tronçon de fibre optique multimodes et/ou à différentes distances de la face de sortie du tronçon de fibre optique multimodes.

Selon un ou plusieurs exemples de réalisation, l'application du déphasage sur chacun des faisceaux élémentaires vise à inscrire à l'extrémité distale du tronçon de fibre optique multimodes une fonction de phase déterminée pour former une figure d'intensité prédéfinie en fonction de la forme de l'objet à imager, ou une figure de speckle.

Selon un ou plusieurs exemples de réalisation, le procédé comprend en outre l'application d'une déviation angulaire sur chacun des faisceaux lumineux élémentaires à l'entrée proximale dudit paquet de fibres optiques monomodes torsadé, ladite déviation angulaire étant déterminée en fonction de la position de la fibre monomode destinée à recevoir ledit faisceau élémentaire dans ledit paquet de fibres optiques monomodes pour optimiser le couplage dans ladite fibre monomode.

Selon un ou plusieurs exemples de réalisation, le procédé est adapté au transport et au contrôle de faisceaux lumineux comprenant des impulsions optiques, et comprend en outre la suppression des retards statiques de vitesse de groupe entre les fibres monomodes dudit paquet de fibres optiques monomodes torsadé au moyen d'un dispositif de contrôle des retards de vitesse de groupe des impulsions lumineuses.

Selon un ou plusieurs exemples de réalisation, le procédé comprend en outre une calibration préalable permettant de déterminer le déphasage à appliquer sur chacun des faisceaux élémentaires en fonction de la fonction de phase recherchée pour le faisceau d'illumination.

Selon un ou plusieurs exemples de réalisation, l'étape de calibration préalable comprend la détermination partielle ou totale d'une matrice de transmission du paquet de fibres optiques monomodes.

Selon un quatrième aspect, la présente description concerne un procédé d'imagerie endo-microscopique sans lentille du coté distal comprenant :
- l'émission de faisceaux lumineux ;
- le transport et le contrôle des faisceaux lumineux au moyen d'un procédé tel que décrit selon le troisième pour l'illumination d'un objet par le faisceau d'illumination ;
- la détection de la lumière renvoyée par l'objet et transmise à travers le guide de lumière, de son extrémité distale à son extrémité proximale.

La lumière renvoyée par l'objet peut être de différente nature en fonction de l'application ; par exemple la lumière renvoyée est la lumière rétrodiffusée, ou la lumière émise, par exemple par un mécanisme de fluorescence.

### BREVE DESCRIPTION DES DESSINS

D'autres avantages et caractéristiques de l'invention apparaîtront à la lecture de la description, illustrée par les figures suivantes :
FIGS. 1A à 1C (déjà décrites), un schéma de principe d'un endoscope dit « sans lentille » selon l'art antérieur, basé sur l'utilisation d'un paquet de fibres monomodes (FIG. 1A); un ensemble de coeurs monomodes d'une fibre multicoeurs selon l'art antérieur (FIG. 1B) ; une figure illustrant le déplacement de la figure d'intensité lors d'une courbure d'un paquet de fibres selon l'art antérieur (FIG. 1C);
FIG. 2, un schéma de principe illustrant un exemple de système d'imagerie endo-microscopique sans lentille selon la présente description ;
FIGS. 3A à 3C, des figures illustrant un paquet de fibres monomodes torsadé selon des exemples de la présente description et FIG. 3D, une figure illustrant la conservation de la figure d'intensité lors d'une courbure d'un paquet de fibres selon la présente description ;
FIG. 4A, un graphique illustrant des conditions de conservation de la fonction de phase pour des rayons de courbure (en abscisse) et des périodes de torsade (en ordonnée) donnés du paquet de fibres optiques torsadé, pour un demi-diamètre du paquet de fibres de 100 µm et pour différentes longueurs de fibres *kP* + *δL,* où *k* est un entier, *P* est la période de torsade, *δL* vaut respectivement *P*/2*, P*/4*, P*/8*, P*/16 ;
FIG. 4B et 4C, des graphiques illustrant la différence de phase additionnelle entre un coeur monomode en périphérie du paquet de fibres et un coeur central, pour des rayons de courbure (en abscisse) et des périodes de torsade (en ordonnée) donnés du paquet de fibres optiques torsadé, pour un demi-diamètre du paquet de fibres de 100 µm (FIG. 4B) et 200 µm (FIG. 4C) et une longueur de fibre *kP* + *P*/2, où *k* est un entier, *P* est la période de torsade ;
FIG. 5, un schéma illustrant l'application d'une déviation angulaire en entrée de fibre pour optimiser le couplage ;
FIGS. 6A et 6B, des courbes illustrant la transmission normalisée en fonction d'une application d'une déviation angulaire en x (FIG. 6A) et en y (FIG. 6B) en fonction de la position du coeur par rapport au coeur central (illustrée sur la FIG. 6C) et FIG. 6D une courbe illustrant la transmission normalisée d'un coeur en fonction de sa distance au coeur central sans application d'une déviation angulaire et avec application d'une déviation angulaire ;

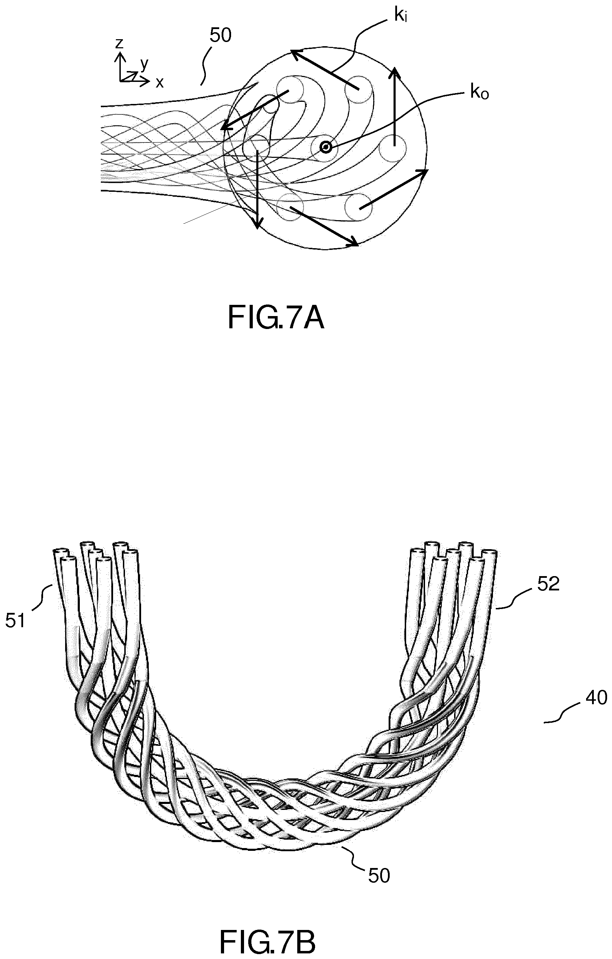

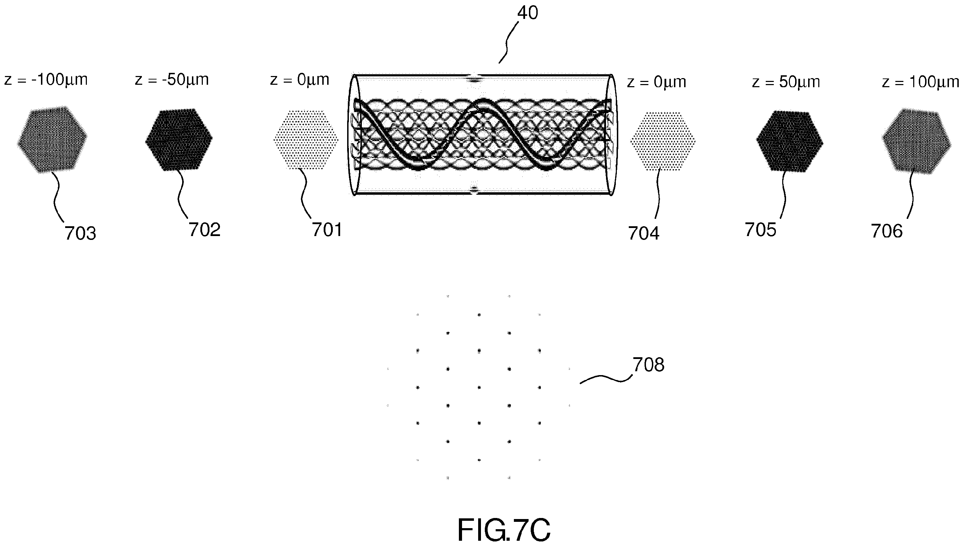

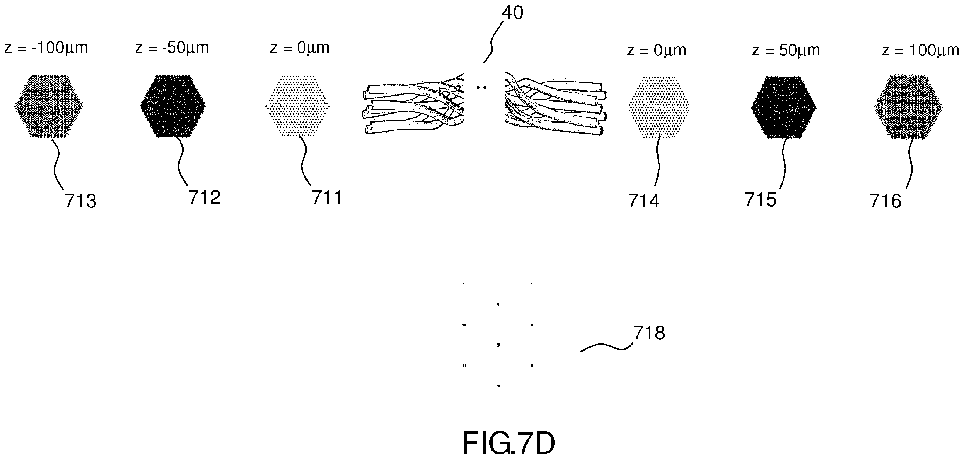

FIG. 7A, un schéma illustrant la déviation angulaire d'un faisceau lumineux en sortie de chaque fibre optique monomode d'un paquet de fibres torsadé ; FIG. 7B, un schéma illustrant un exemple de guide de lumière selon la présente description comprenant un paquet de fibres optiques monomodes torsadé et aux extrémités, un tronçon de fibres non torsadé ; FIGS. 7C et 7D des schémas illustrant des figures d'intensité du champ électromagnétique à différentes distances en amont et en aval du paquet de fibres optiques torsadé dans le cas d'un paquet de fibres optiques torsadé sans tronçons non torsadés (FIG. 7C) et avec tronçons non torsadés (FIG. 7D) ;
FIGS. 8C et 8D, des images comparatives d'une mire (FIG. 8B) obtenues avec un paquet de fibre non courbé et courbé (FIG. 8A);
FIG. 9, une figure d'interférence permettant d'illustrer la conservation de la phase entre une fibre périphérique et une fibre centrale, avec une fibre torsadée ;
FIGS. 10A, 10B, des schémas illustrant respectivement le retard de groupe (GDD) intrinsèque dans un paquet de fibres optiques monomodes torsadé selon un exemple de la présente description et la variation du retard de groupe entre le paquet de fibre torsadé courbé et non courbé.

### DESCRIPTION DETAILLEE

Dans la description détaillée qui suit, seuls certains modes de réalisation sont décrits en détail pour assurer la clarté de l'exposé mais ces exemples ne visent pas à limiter la portée générale des principes ressortant de la présente description.

Les différents modes de réalisation et aspects décrits dans la présente description peuvent être combinés ou simplifiés de multiples manières. En particulier, les étapes des différents procédés peuvent être répétées, interverties, exécutées en parallèle, sauf précision contraire.

Lorsque dans la présente description, il est fait référence à des étapes de calcul ou traitement pour la mise en oeuvre notamment d'étapes de procédés, il est entendu que chaque étape de calcul ou traitement peut être mis en oeuvre par logiciel, hardware, firmware, microcode ou toute combinaison appropriée de ces technologies. Lorsqu'un logiciel est utilisé, chaque étape de calcul ou traitement peut être mise en oeuvre par des instructions de programme d'ordinateur ou du code logiciel. Ces instructions peuvent être stockées ou transmises vers un support de stockage lisible par un ordinateur (ou unité de contrôle) et/ou être exécutées par un ordinateur (ou unité de contrôle) afin de mettre en oeuvre ces étapes de calcul ou traitement.

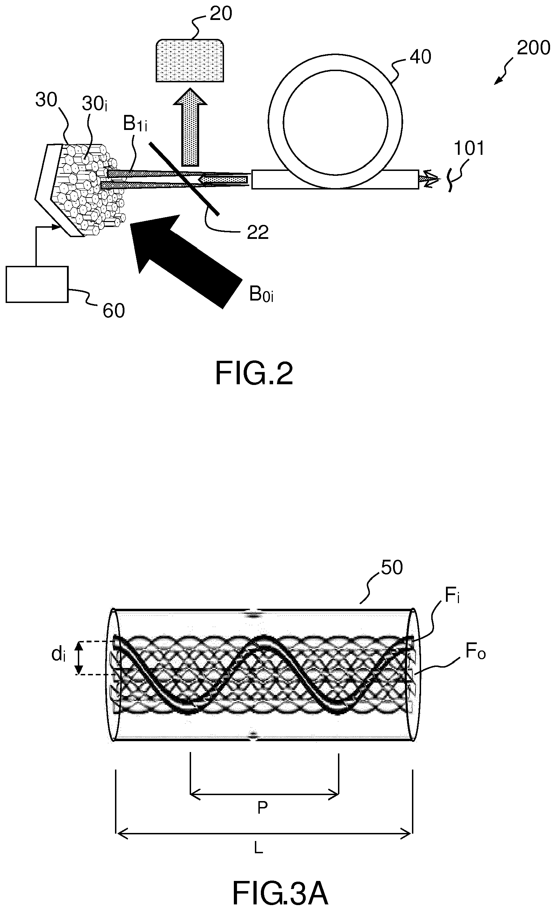

La FIG. 2 illustre de façon schématique un exemple de système 200 d'imagerie endo-microscopique « sans lentille » avec un dispositif de transport et de contrôle de faisceaux lumineux selon la présente description, adapté pour l'imagerie d'un objet référencé 101 sur la FIG. 2.

Le système d'imagerie endo-microscopique 200 comprend une source lumineuse (non représentée sur la FIG. 2) adaptée pour l'émission de faisceaux lumineux B₀ᵢ, les faisceaux lumineux pouvant comprendre des impulsions lumineuses dans le cas d'une application à l'imagerie non linéaire. Ainsi, la source lumineuse comprend par exemple une source laser et, si nécessaire, un système optique de grandissement et de collimation des faisceaux lumineux émis.

Le système d'imagerie endo-microscopique 200 comprend par ailleurs un dispositif pour le transport et le contrôle des faisceaux lumineux émis par ladite source lumineuse afin d'éclairer l'objet 101 selon une figure d'intensité choisie, par exemple un ou plusieurs points de focalisation, par exemple balayé(s) dans le champ, ou d'autres formes, suivant les applications. Le dispositif pour le transport et le contrôle des faisceaux lumineux comprend généralement un guide de lumière 40 comprenant un paquet de fibres optiques monomodes (ou « MCF ») torsadé, comme cela sera expliqué plus en détails par la suite, et un dispositif optique de contrôle de la phase agencé du côté d'une extrémité proximale du premier guide de lumière, comprenant notamment un modulateur spatial de lumière 30.

Le paquet de fibres optiques monomodes torsadé peut comprendre un ensemble de fibres optiques monomodes individuelles, typiquement une centaine à quelques dizaines de milliers de fibres, rassemblées sous forme d'un faisceau de fibres, ou peut comprendre un ensemble de coeurs monomodes d'une fibre multicoeur, de préférence au moins une centaine. La fibre multicoeur est par exemple une fibre à double gaine.

Le guide de lumière 40 peut comprendre d'autres éléments, par exemple tout élément utile à la réalisation du guide, comme des éléments de protection, de façon connue par l'homme du métier. Dans le cas d'une fibre multicoeur à double gaine, une gaine peut être une gaine multimode, adaptée à la propagation du flux lumineux rétrodiffusé par l'objet.

Avantageusement, le couplage entre les coeurs monomodes du paquet de fibres optiques monomodes est inférieur à -20 dB/m, avantageusement inférieur à -30 dB/m, ce qui permet le transport et le contrôle des faisceaux optiques sur une grande longueur du paquet de fibres, tout en offrant la possibilité de compenser les effets de déphasage inter-coeur.

La longueur des fibres monomodes du paquet de fibres est adaptée à l'application, par exemple à la longueur requise pour un endo microscope. Typiquement, la longueur des fibres monomodes du paquet de fibres est comprise entre 30 cm et 3 m.

Le dispositif optique de contrôle de la phase est agencé du côté de l'extrémité proximale du paquet de fibres optiques monomodes et comprend le modulateur spatial de lumière 30 adapté pour l'application d'un déphasage sur chacun des faisceaux lumineux élémentaires B₀ᵢ et une unité de contrôle 60 du modulateur spatial de lumière permettant l'application d'un déphasage sur chacun des faisceaux élémentaires pour l'inscription à l'extrémité distale du guide de lumière 40 d'une fonction de phase déterminée. Le modulateur spatial de lumière 30 peut comprendre par exemple un miroir déformable segmenté ou à membrane, pour un fonctionnement en réflexion, ou une matrice de cristaux liquides, pour un fonctionnement en réflexion ou en transmission. Dans l'exemple de la FIG. 2, chaque surface élémentaire 30i du modulateur spatial de lumière 30 permet d'appliquer à un faisceau lumineux élémentaire B₀ᵢ un déphasage donné Φ₁(i) pour former un faisceau élémentaire B₁ᵢ destiné à entrer dans une fibre optique Fᵢ du guide de lumière 40.

Par exemple, comme cela est illustré sur la FIG. 2, la fonction de phase Φ₁(i) est déterminée pour former à l'extrémité distale du guide de lumière 40, en champ lointain, un faisceau convergent pour former un point de focalisation dans un plan situé à une distance donnée de la face de sortie du guide de lumière 40 et comprenant l'objet d'intérêt 101.

Le dispositif pour le transport et le contrôle des faisceaux lumineux est dit « sans lentille » car il ne comprend pas de lentille du côté distal, c'est-à-dire du côté de l'émergence des faisceaux lumineux, la phase étant contrôlée par le dispositif de contrôle de la phase agencé du côté d'une extrémité proximale du dispositif.

Une calibration préalable du dispositif peut être prévue pour déterminer le déphasage à appliquer sur chacun des faisceaux élémentaires en fonction de la fonction de phase recherchée pour le faisceau d'illumination. Cette étape de calibration préalable peut comprendre la détermination d'une matrice de transmission du paquet de fibres optiques monomodes. Dans le cas d'un paquet de fibres optiques monomodes avec un faible couplage entre coeurs, la matrice de transmission peut être une matrice diagonale reliant les modes fondamentales de chaque coeur en entrée et en sortie. Ainsi, il suffirait de mesurer uniquement les déphasages relatifs acquis par les faisceaux élémentaires après avoir traversé le paquet de fibres optiques et cela à une seule longueur d'onde (typiquement la longueur d'onde centrale de la source laser lumineuse utilisée).

Selon un exemple de réalisation, le système d'imagerie 200 peut comprendre aussi des moyens (non représentés sur la FIG. 2) de focalisation des faisceaux lumineux élémentaires B₀ᵢ sur les éléments 30i du modulateur spatial de lumière 30. Les moyens de focalisation des faisceaux lumineux élémentaires B₀ᵢ comprennent par exemple une matrice de micro lentilles ou un modulateur spatial de lumière, par exemple une matrice de cristaux liquides formant un pavage bidimensionnel de réseaux ayant des phases quadratiques, simulant ainsi une matrice de microlentilles.

Le système d'imagerie endo-microscopique 200 comprend également une voie de détection de la lumière rétrodiffusée par l'objet 101 et transmise à travers le guide de lumière 40 de son extrémité distale à son extrémité proximale. Sur l'exemple de la FIG. 2, la voie de détection comprend une lame séparatrice de faisceaux 22, un détecteur 20 et éventuellement un objectif (non représenté sur la FIG. 2) pour focaliser la lumière rétrodiffusée sur une surface de détection du détecteur 20, ainsi qu'une unité de traitement (non représenté) des signaux issus du détecteur 20.

Un exemple de paquet de fibres optiques monomodes torsadé 50 selon la présente description est illustré sur les FIGS 3A à 3C.

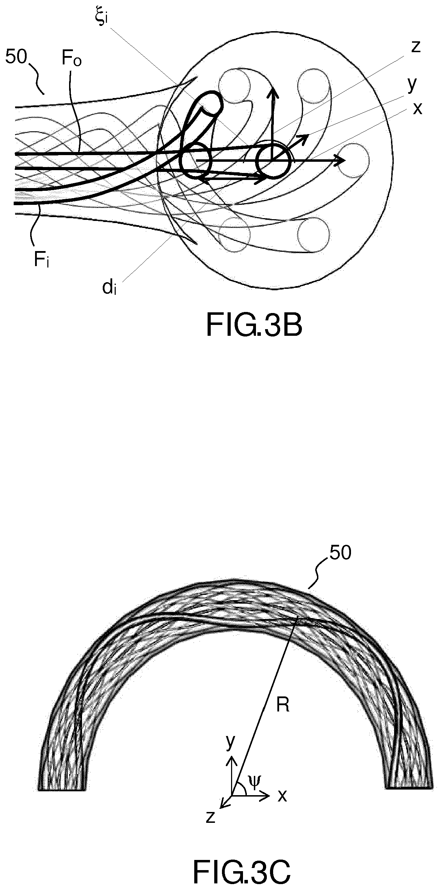

Un paquet de fibres optiques (ou coeurs) monomodes torsadé 50 comprend généralement un coeur central Fo rectiligne et un ensemble de coeurs Fi enroulés autour du coeur central Fo de façon hélicoïdale. La longueur L du paquet de fibres optiques monomodes correspond à la longueur du coeur central Fo. Le paquet de fibres optiques monomodes torsadé 50 comprend une période de torsade P. comme illustré sur la FIG. 3B, chaque coeur Fi peut être caractérisé par une distance dᵢ par rapport au coeur central Fo et un angle ξᵢ défini par l'angle azimutal du coeur Fᵢ sur la face d'entrée de la MCF (FIG. 3B) pour un coeur donné, dᵢ et ξᵢ sont constants. On peut définir un demi-diamètre d du paquet de fibres optiques monomodes comme la distance du coeur central au coeur le plus éloigné. Typiquement, le demi-diamètre d est compris entre 100 µm et 500 µm, avantageusement entre 100 µm et 300 µm, avantageusement entre 100 µm et 200 µm.

Lorsque le paquet de fibres optiques monomodes 50 subit une courbure (FIG. 3C), on définit localement un rayon de courbure R de la courbure, correspondant au rayon de courbure du coeur central, à une coordonnée polaire ψ défini sur la FIG. 3C par rapport au centre de courbure. Dans la suite de la description, le rayon de courbure minimal du paquet de fibres optiques monomodes 50 correspond à la courbure maximale locale du paquet de fibres optiques monomodes 50, en opération.

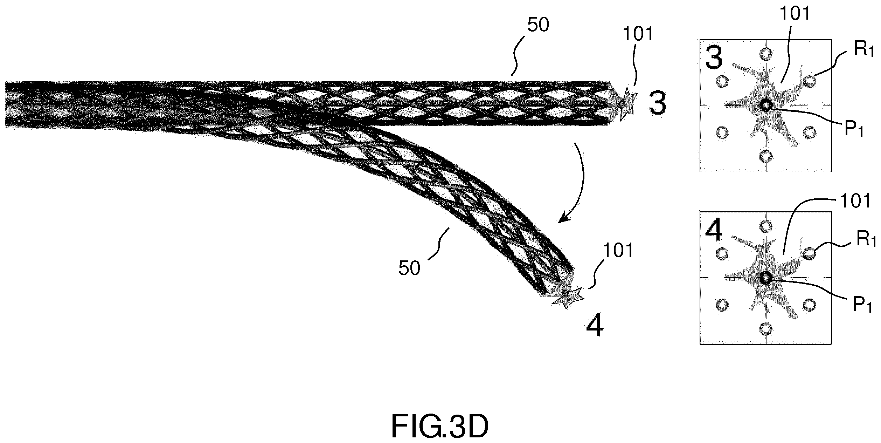

La FIG. 3D illustre la conservation de la figure d'intensité lors d'une courbure d'un paquet de fibres selon l'art antérieur. En comparaison avec un paquet de fibres non torsadé selon l'art antérieur (voir FIG. 1C), les déposants ont montré que l'on pouvait obtenir une excellente conservation de la fonction de phase à l'extrémité distale du paquet de fibres, et ainsi une conservation de la figure d'intensité.

Les déposants ont montré que l'on peut définir une période de torsade P du paquet de fibres optiques monomodes en fonction du rayon de courbure minimal en opération pour conserver la figure d'intensité à l'extrémité distale du guide de lumière 40.

Plus précisément, les déposants ont montré que si la longueur L du paquet de fibres est telle que L/P= k (avec k entier), c'est-à-dire s'il y a un nombre entier de fois la période de torsade sur la longueur du paquet de fibres, alors le paquet de fibres torsadé est insensible à la courbure et le déphasage additionnel entre les coeurs est égal à zéro. Il est ainsi possible pour le paquet de fibres optiques de supporter des rayons de courbure aussi petits que quelques millimètres sans modification de la figure d'intensité en sortie distale du paquet de fibres.

Dans ce cas, la limite basse Pₘᵢₙ à la valeur de la période correspond à la courbure maximale que peut subir un coeur monomode sans entrainer de pertes optiques supérieures à une valeur de seuil donnée. Ainsi par exemple, pour un seuil de pertes optiques fixé à 1dB/m (c'est-à-dire que 80% de l'énergie est transmise au bout de 1m de fibre), les déposants ont montré que la période pouvait être avantageusement supérieure à 2,5 mm.

Les déposants ont également montré que si L/P n'est pas entier, c'est-à-dire L/P= k + *δL* (avec k entier), le déphasage additionnel Δ(ΔΦ) entre les coeurs subissant une courbure du paquet de fibres n'est pas zéro, mais une fonction de dᵢ, ξᵢ, P, R (rayon minimal) et *δL.*

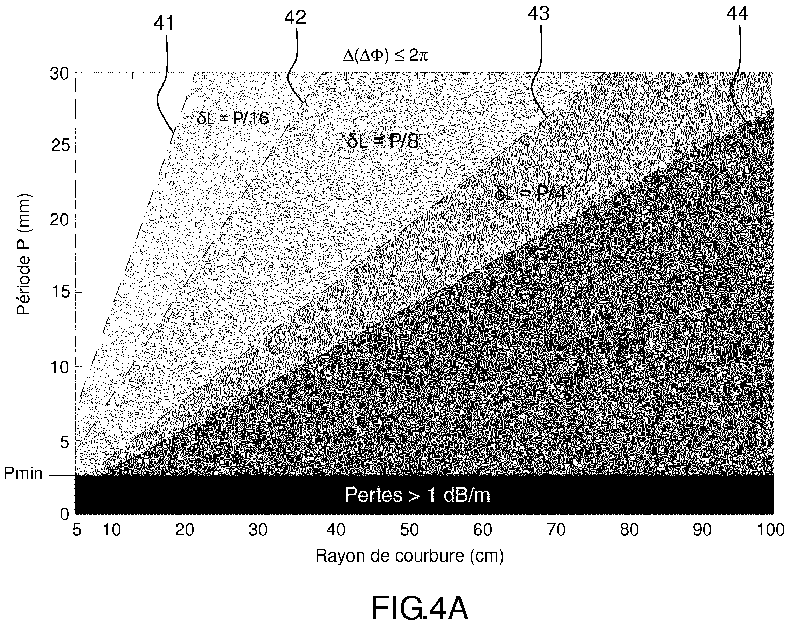

La FIG. 4A illustre ainsi pour différentes valeurs de *δL,* la relation (représentée par une ligne interrompue) entre la période de torsade P et le rayon de courbure minimal pour avoir un déphasage additionnel entre le coeur central et un coeur situé à la périphérie du paquet de fibres de 2π. Pour chaque valeur de *δL,* les valeurs en-dessous de la ligne interrompue correspondante correspondent à des valeurs Δ(ΔΦ) ≤ 2π. Ainsi, la ligne interrompue 41 correspond à une valeur Δ(ΔΦ) = 2π pour *δL* = ±P/16, la ligne interrompue 42 correspond à une valeur Δ(ΔΦ) = 2π pour *δL* = ±P/8, la ligne interrompue 43 correspond à une valeur Δ(ΔΦ) = 2π pour *δL* = ±P/4, la ligne interrompue 44 correspond à une valeur Δ(ΔΦ) = 2π pour *δL* = ±P/2.

Les courbes sont calculées en utilisant les équations décrivant la propagation de la lumière dans une fibre torsadée et décrites par exemple dans Napiorkowski et al. « Rigorous simulations of a helical fiber by the use of transformation optics formalism », Opt. Express 22(19), 2014). Plus précisément, pour le calcul des courbes de la FIG. 4A, on considère une distance *d* (ou demi-diamètre) entre le coeur central et un coeur situé à la périphérie de 100 µm et on se place dans un cas de courbure du paquet de fibres qui introduit un déphasage maximal entre le coeur central et le coeur situé à la périphérie du paquet de fibres.

On observe sur cette figure que plus *δL* est petit moins il sera nécessaire de « torsader » le paquet de fibres optiques pour un rayon de courbure minimal donné. Autrement dit, on pourra choisir une période de torsade suffisamment grande et s'éloigner de la valeur minimum de la période Pₘᵢₙ déterminée pour ne pas dépasser un seuil de pertes optiques déterminé.

Par exemple, dans l'exemple illustré sur la FIG. 4A, si l'on se place dans le pire des cas (*δL* = P/2) et si l'on suppose, en opération, une courbure maximale du paquet de fibres correspondant à un rayon de courbure de 30 cm, on pourra choisir une période de torsade d'environ 8 mm pour conserver la fonction de phase à l'extrémité distale du guide de lumière. On observe qu'il sera difficile dans cette hypothèse de conserver la fonction de phase sans pertes de lumière si le rayon de courbure est aussi petit que 10 cm. Par contre, avec des valeurs *δL* plus faibles, on pourra conserver la fonction de phase sans perte de lumière et il sera possible de choisir des périodes de torsade plus grandes.

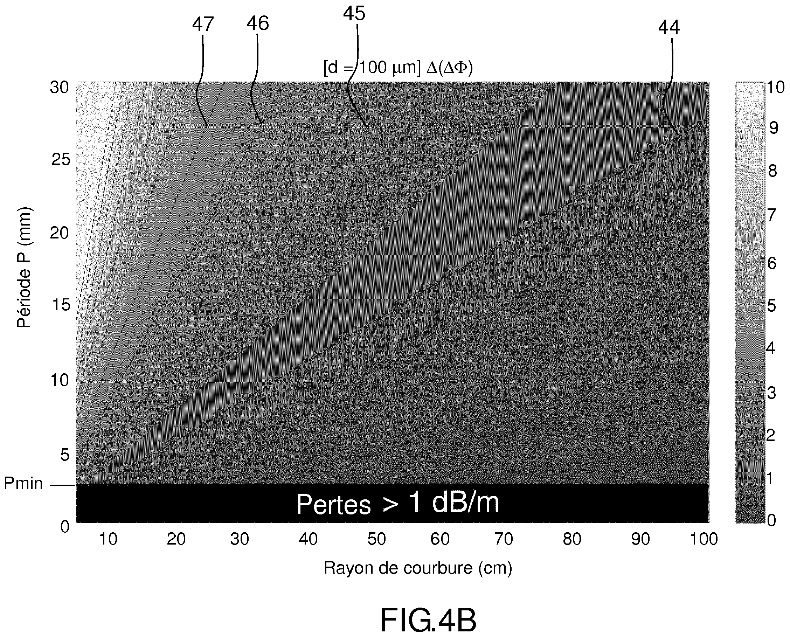

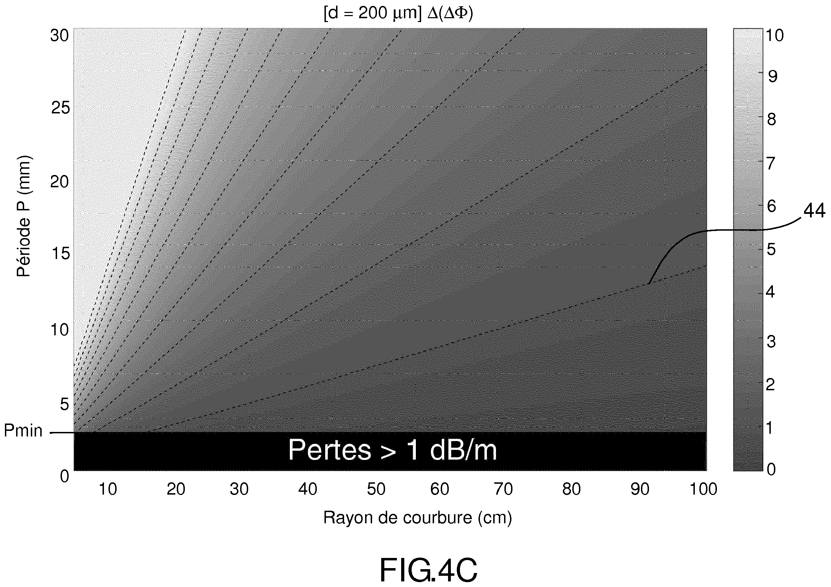

Les FIGS 4B et 4C représentent le déphasage additionnel Δ(ΔΦ) respectivement pour une distance *d* entre le coeur central et un coeur situé à la périphérie est de 100 µm (diamètre du paquet de fibres de 200 µm) et une distance *d* entre le coeur central et un coeur situé à la périphérie est de 200 µm (diamètre du paquet de fibres de 400 µm). Sur ces figures, on suppose *δL* = P/2 (pire des hypothèses). La ligne en pointillé 44 correspond comme précédemment à une valeur Δ(ΔΦ) = 2π. les autres lignes 45, 46, 47, etc. correspondent à des valeurs de déphasage additionnel Δ(ΔΦ) de 4π, 6π, 8π, etc. En pratique, on choisira une valeur de période de torsade pour conserver un déphasage additionnel inférieure à 2π lorsque le paquet de fibres subit en opération une courbure maximale correspondant à un rayon de courbure minimal donné.

On observe en comparant les figures 4B et 4C que pour un rayon de courbure donné, on pourra choisir une période de torsade plus grande, et donc plus éloignée de la période minimale déterminée pour ne pas dépasser un seuil de pertes optiques déterminé, avec des paquets de fibres optiques de plus faibles diamètres.

En pratique, les diamètres des paquets de fibres optiques sont généralement inférieurs à 400 µm ce qui montre la faisabilité de notre méthode pour conserver la fonction de phase à l'extrémité distale du paquet de fibres optiques.

Un paquet de fibres optiques monomodes torsadé selon la présente description peut être fabriqué selon des moyens connus, décrits par exemple dans P. S. J. Russell et al. ("Helically twisted photonic crystalfibres" Philosophical Transactions of the Royal Society A: Mathematical, Physical and Engineering Sciences 375 (2017)).

Selon un premier exemple, une rotation de la préforme est effectuée au moment du tirage du paquet de fibres. Cette technique utilise un moteur rotatif (qui tourne à quelques milliers de tours par minute) et un joint rotatif. La période de torsade est ainsi égale à la vitesse de tirage (en m/s) divisée par la fréquence de rotation (en Hz) de la préforme. Avec cette approche, il est possible d'obtenir des périodes de torsade de quelques millimètres sur des longueurs de 100 m de fibre.

Selon un deuxième exemple, la torsion en rotation est effectuée après le tirage du paquet de fibres. Pour cela on monte le paquet de fibres entre un moteur rotatif et un support fixe, on utilise ensuite un laser CO2 qui, focalisé sur le paquet de fibres, fait fondre la silice. Un contrôle de la tâche de focalisation du laser CO2, de son temps d'exposition, et de son déplacement sur le paquet de fibres en rotation permet de contrôler les paramètres de la torsion. Cette technique permet de faire varier la période de torsade le long du paquet de fibres.

Les déposants ont également montré comment il était possible d'améliorer le couplage dans les coeurs monomodes d'un paquet de fibres optiques torsadé.

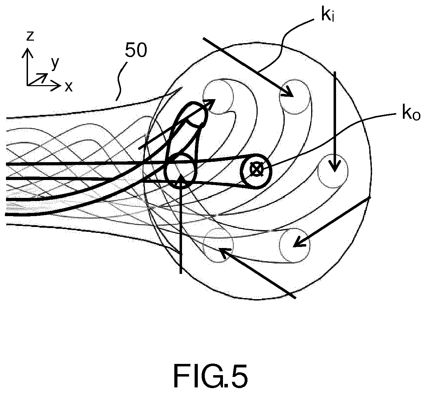

La FIG. 5 montre la face d'entrée de la MCF torsadée 50. Le mode fondamental du coeur central se propage parallèlement avec l'axe de la MCF ; en d'autres mots son vecteur de propagation k₍ᵢ₌₀₎ (représenté par une croix sur la fibre centrale) est parallèle avec l'axe de la MCF. Le faisceau élémentaire qui entre dans le coeur central avec la plus grande efficacité de couplage est celui dont le vecteur de propagation est égal à ko, soit un faisceau élémentaire qui se propage, lui aussi, parallèlement à l'axe de la MCF. La situation est différente pour les coeurs excentrés : Du fait de l'hélicité de ces coeurs, le mode fondamental dans ces coeurs se propage avec un angle par rapport à l'axe de la MCF ; en d'autres termes, le vecteur de propagation kᵢ pour un coeur excentré n'est pas parallèle à l'axe de la MCF. Pour assurer un couplage optimal, les déposants ont montré qu'il était avantageux que chaque faisceau élémentaire qui entre dans un coeur excentré présente un vecteur de propagation égal à kᵢ et ainsi présente un angle avec l'axe de la MCF.

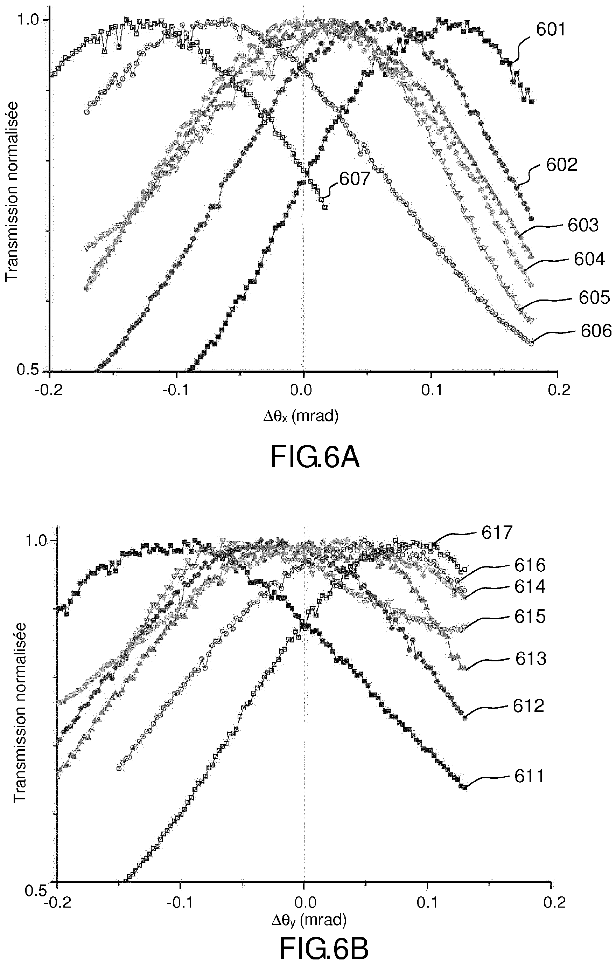

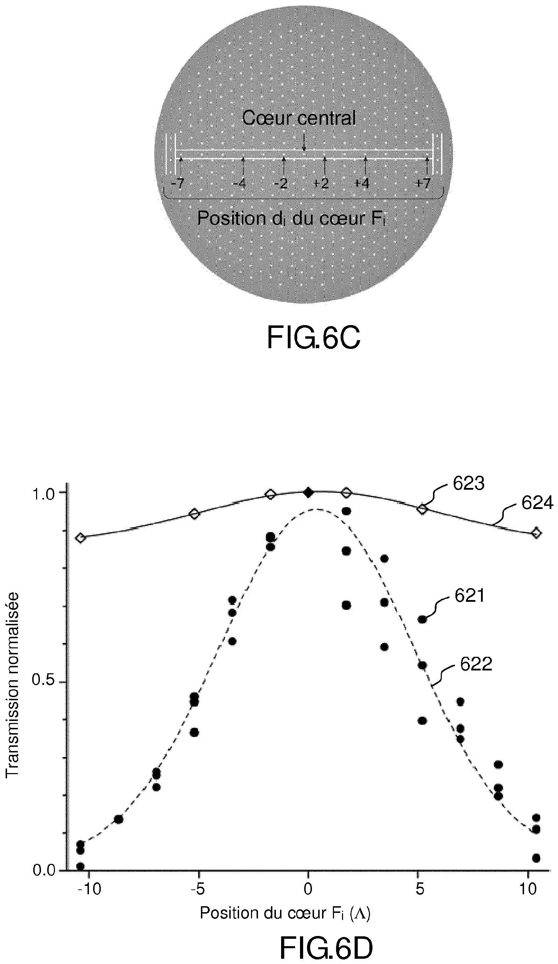

Plus précisément, les déposants ont fait des mesures montrant l'influence de la déviation angulaire Δθₓ⁽ⁱ⁾(FIG. 6A) et de la déviation angulaire Δθ_{y}⁽ⁱ⁾ (FIG. 6B) sur la transmission normalisée de coeurs monomodes Fᵢ situé à différentes distances dᵢ du coeur central. La FIG. 6C montre le positon du coeur Fᵢ exprimée en nombre de fois la distance A entre deux coeurs voisins de la MCF (ou « pitch ») de la MCF. Ainsi, la position +7 correspond à un coeur Fᵢ situé à une distance dᵢ égale à 7A du coeur central où A est le pitch de la MCF.

Sur la FIG. 6B, les courbes 601 - 607 correspondent respectivement à des coeurs situés à des distances du coeur central égales à -7A, -4A, -2A, 0, +2A, +4A, +7A.

Sur la FIG. 6C, les courbes 611 - 617 correspondent respectivement à des coeurs situés à des distances du coeur central égales à -7A, -4A, -2A, 0, +2A, +4A, +7A.

Ces courbes illustrent que la transmission normalisée est rendue maximale en appliquant une déviation angulaire Δθₓ⁽ⁱ⁾, Δθy⁽ⁱ⁾ qui dépend de la distance du coeur Fᵢ au coeur central Fo.

La FIG. 6D représente la transmission normalisée d'un coeur Fᵢ en fonction de sa distance au coeur central Fo (en nombre de fois la distance A entre deux coeurs voisins de la MCF (ou «pitch»)) sans application d'une déviation angulaire (courbes 621, 622) et avec application d'une déviation angulaire (courbes 623, 624). On constate que l'application d'une déviation angulaire permet d'augmenter significativement le couplage dans les fibres optiques dont la distance par rapport au centre est grande. Par exemple pour un coeur situé à 10 A du coeur central une déviation angulaire de 0.15mrad suivant x et y permet d'augmenter le couplage de 80% (on passe de 10% sans déviation angulaire à 90% avec déviation angulaire)

A noter que c'est la partie transverse du vecteur de propagation qui est conservée à travers l'interface entre espace libre et la MCF torsadée (du fait de la conservation du moment). En termes d'angles de propagation, les angles en espace libre sont un facteur n plus grands que dans la MCF, n étant l'indice de réfraction du verre constituant la MCF.

Ainsi, il avantageux de prévoir l'application d'une déviation angulaire θi sur chacun des faisceaux élémentaires B₁ᵢ à l'entrée proximale dudit paquet de fibres optiques monomodes torsadé (voir FIG. 2), ladite déviation angulaire θi étant déterminée en fonction de la position du coeur monomode Fᵢ destiné à recevoir le faisceau élémentaire dans ledit paquet de fibres optiques monomodes, et ce afin d'améliorer le couplage dans le coeur monomode.

Même si on applique une déviation angulaire θi sur chacun des faisceaux élémentaires B₁ᵢ à l'entrée proximale dudit paquet de fibres optiques monomodes torsadé comme précédemment décrit, on observe également du fait de la torsade une déviation angulaire en sortie du paquet de fibres optiques.

La FIG. 7A montre représente ainsi une face de sortie de la MCF torsadée 50. Le mode fondamental du coeur central se propage parallèlement avec l'axe de la MCF ; en d'autres mots son vecteur de propagation k₍ᵢ₌₀₎ (représenté par un point sur la fibre centrale) est parallèle avec l'axe de la MCF. Le faisceau élémentaire qui sort du coeur central avec la plus grande efficacité de couplage est celui dont le vecteur de propagation est égal à ko, soit un faisceau élémentaire qui se propage, lui aussi, parallèlement à l'axe de la MCF. La situation est différente pour les coeurs excentrés : du fait de l'hélicité de ces coeurs, le mode fondamental dans ces coeurs se propage avec un angle par rapport à l'axe de la MCF ; en d'autres termes, le vecteur de propagation kᵢ pour un coeur excentré n'est pas parallèle à l'axe de la MCF.

Une façon d'obtenir un couplage optimal à l'extrémité distale et/ou proximale du guide de lumière est illustrée sur la FIG. 7B.

Dans cet exemple, le guide de lumière 40 comprend un paquet de fibres optiques monomodes torsadé 50 et aux extrémités, des tronçons de fibres, respectivement référencés 51 et 52, présentant une variation de la période de la torsade allant jusqu'à l'infini aux extrémités ; autrement dit, à l'interface du tronçon avec l'espace libre, les fibres optiques monomodes sont parallèles et le paquet de fibres n'est plus torsadé.

Les déposants ont montré que l'on peut réaliser une MCF comprenant un court tronçon proximal non-torsadé 51 qui évolue en un long tronçon torsadé 50 qui évolue finalement vers un court tronçon distal non-torsadé 52. Les transitions entre MCF torsadée et MCF non-torsadée se font avantageusement de manière graduelle, c'est-à-dire P incrémente ou décrémente de manière continue, ce qui assure une efficacité de transmission de 100 % à travers la zone de transition. Cf Fig. 7B. Les tronçons non torsadés en partie distale et proximale ont une longueur par exemple inférieure ou égale à 1cm.

Le guide de lumière représenté sur la FIG. 7B peut être fabriqué en chauffant localement la MCF entre deux régions grâce à un laser CO2 ; lorsque le verre est fondu, on applique une torsion contraire à celle de la MCF ; il en résulte une partie non-torsadée ; la MCF est alors clivé à l'endroit non-torsadé, ce qui donne une extrémité non-torsadée. Le procédé est répété pour l'autre extrémité de la MCF.

Les FIGS 7C et 7D illustrent ainsi des figures représentant des figures en intensité du champ électromagnétique à différentes distances en amont et en aval du paquet de fibres optiques torsadé dans le cas d'un paquet de fibres optiques torsadé sans tronçons non torsadés (FIG. 7C) et avec tronçons non torsadés (FIG. 7D).

Dans le cas de la FIG. 7C, on suppose une application de déviations angulaires sur chacun des faisceaux élémentaires à l'entrée proximale du paquet de fibres optiques monomodes torsadé, comme précédemment décrit. L'application de déviations angulaires est visible sur les figures d'intensité 701 au niveau de la face d'entrée (z = 0 µm) et sur les figures en intensité 702, 703 respectivement à z = - 50 µm et z = - 100 µm qui montrent que le champ a « tourné » pour atteindre la face d'entrée. Autrement dit, le champ est arrivé sous un angle donné qui n'est pas perpendiculaire à la face d'entrée. Cet angle est différent en fonction de la position du coeur (ξᵢ) et sa distance au coeur central. En sortie, du fait des déviations angulaires subis par les faisceaux élémentaires qui se propagent dans le paquet de fibres optiques torsadé, on observe que le champ électromagnétique se propage en tournant (figures en intensité 704, 705, 706 correspondant respectivement à z = 0 µm (face de sortie) z = 50 µm et z = 100 µm). Il en résulte un mauvais recouvrement dans le champ lointain entre les faisceaux issus des coeurs monomodes, ce qui conduit à un "étalement" (augmentation du champ de vue) et une diminution de l'intensité de la PSF dans sa partie centrale (figure en intensité 708).

Dans le cas d'un guide de lumière 40 comprenant un paquet de fibres optiques torsadé et deux tronçons avec variation de la période de torsade pour aboutir à des extrémités non torsadées (FIG. 7D), on observe que la figure en intensité est stable en sortie du guide (figures en intensité 714, 715, 76 correspondant respectivement à z = 0 µm, z = 50 µm et z = 100 µm) et conduit à une PSF non étalée avec une forte intensité dans la partie centrale (figure en intensité 718). A noter que dans le cas de la FIG. 7D on a prévu un tronçon de paquet de fibres non torsadé en entrée, ce qui permet de s'affranchir de l'application de déphasages angulaires sur les faisceaux élémentaires à l'entrée proximale du paquet de fibres. Cela explique que les figures en intensité 711, 712, 713 correspondant respectivement à z = 0 µm, z = - 50 µm et z = - 100 µm sont stables. On aurait pu prévoir un tronçon de paquet de fibres non torsadé seulement en sortie et l'application de déphasages angulaires sur les faisceaux élémentaires à l'entrée proximale du paquet de fibres.

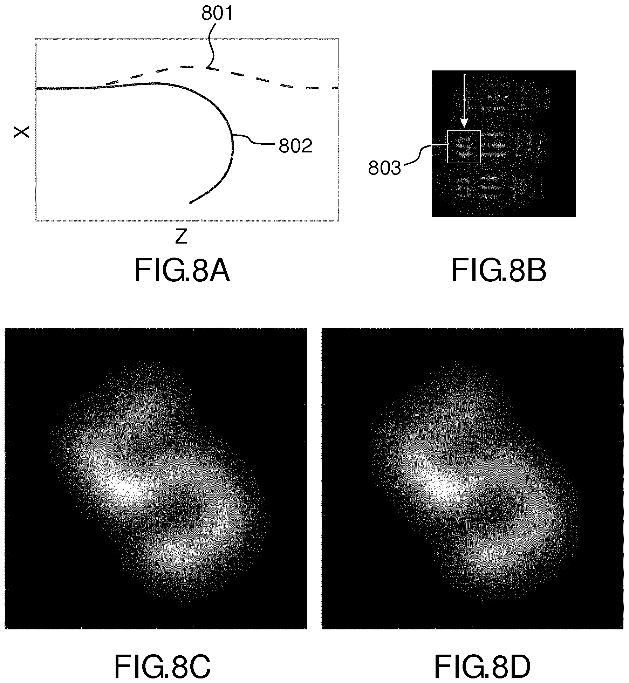

Les FIGS. 8A à 8D montrent des images comparatives obtenues avec un paquet de fibres (complètement torsadé). Le montage utilisé pour obtenir les images est celui représenté sur la FIG.2, avec un détecteur agencé du côté distal du paquet de fibres optiques (images en transmission). Dans l'hypothèse d'une fibre torsadée sans courbure (801) et avec courbure (802) (FIG. 8A). L'imagerie est réalisée suivant la modalité décrite précédemment qui consiste à ajuster les déphasages relatifs des différents coeurs d'une fibre MCF de façon à obtenir un point de focalisation à l'extrémité de la MCF puis à balayer ce point de focalisation sur un échantillon. L'échantillon est constitué ici du chiffre '5' sélectionné dans une mire USAF négative (FIG. 8B) de telle sorte que l'image est reconstruite à partir de l'intensité transmise pour chaque point de l'échantillon.

La Fig. 8C montre une image obtenue en transmission avec le paquet de fibres pour une position quasi linéaire du paquet de fibres (801, FIG. 8A) et pour une courbure de 135° (802, FIG. 8A). L'image est quasiment identique dans les deux cas, ce qui démontre que le point de focalisation bouge très peu (d'une distance inférieure à une PSF ou de l'ordre d'une PSF) dans le champ de vue lorsque le paquet de fibres est courbé.

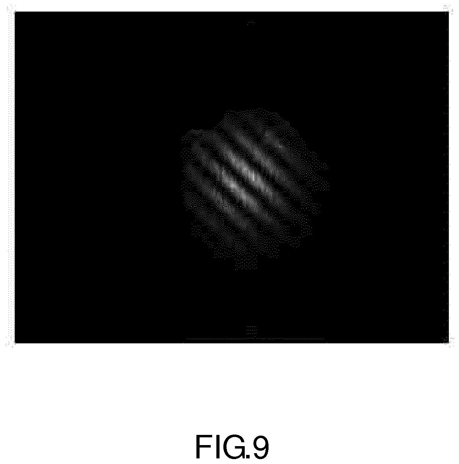

La FIG. 9 illustre une figure d'interférence illustrant la conservation de la phase entre une fibre périphérique et une fibre centrale, avec une fibre torsadée.

Plus précisément, afin d'évaluer la stabilité de la phase relative des coeurs lors d'une courbure pour un paquet de fibres torsadé, les déposants ont mené des expériences d'interférométrie entre le coeur central et les coeurs périphériques. Lorsque la lumière est injectée dans la fibre centrale et dans une fibre périphérique on obtient des franges d'inférences en partie distale de la MCF caractéristiques de l'interférence à deux ondes (FIG. 9). De façon remarquable, cette figure d'interférence ne change pas quand le paquet de fibres torsadé est courbé.

Les déposants ont montré que les méthodes et dispositifs selon la présente description peuvent également être appliqués au transport et au contrôle de faisceaux lumineux en imagerie non linéaire, le dispositif étant adapté à la transmission d'impulsions courtes.

Cependant, dans le cas de manipulations d'impulsions ultra-courtes, on observe un retard de vitesse de groupe (ou GDD pour « group delay dispersion ») qui représente le retard subi entre des impulsions lumineuses voyageant dans les différents coeurs du paquet de fibres. Il existe deux types de retard de vitesse de groupe, le GDD dit 'statique' qui est associé aux retards de vitesse de groupe entre coeurs pour une fibre positionnée linéairement, ce retard étant lié aux variations de chemins optiques résiduels existants entre les différents coeurs du paquet de fibres et le GDD dit 'dynamique' qui est associé au retard de vitesse de groupe entre coeurs quand le paquet de fibres subit une courbure.

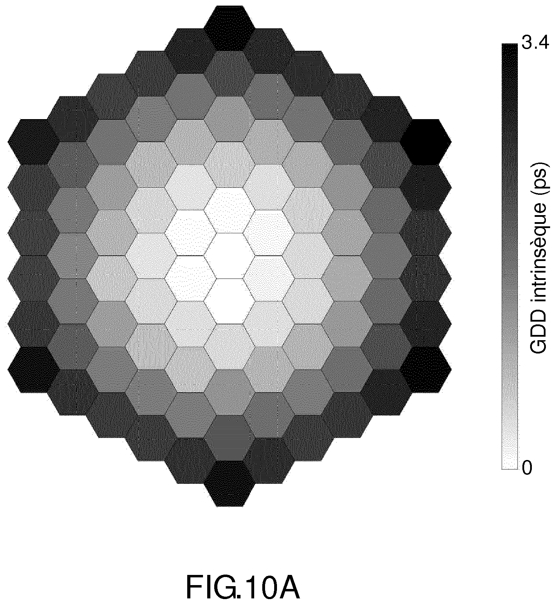

Dans le cas d'un paquet de fibres torsadé comme dans la présente description, la courbure génère un GDD intrinsèque illustré sur la FIG. 10A.

Plus précisément, La FIG. 10A illustre le GDD mesuré pour un paquet de fibres de 300 mm de long torsadé, une période de torsade P = 8.2 mm, chaque hexagone représentant un coeur unique ; la distance entre les centres des hexagones adjacents est A = 15.9 µm. Ne sont montrés ainsi que les coeurs situés dans un rayon de 5^{∗}15.9 = 80 µm du centre de paquet de fibres. Chaque hexagone représente un coeur du paquet de fibres et le GDD associée à ce coeur. On observe un fort délai de vitesse de groupe sur le paquet de fibre torsadé. Ceci s'explique par la différence des longueurs effectives des coeurs à l'intérieur du paquet. Le chemin optique des coeurs centraux est court alors qu'il est beaucoup plus long pour les fibres périphériques, d'où les différences des chemins optiques et donc le GDD observé.

On peut compenser le GDD observé sur la FIG. 10A avec un dispositif de contrôle de la vitesse de groupe des impulsions lumineuses dans le paquet de fibres optiques monomodes (ou GDC pour « Group Delay Control »), comme cela est décrit dans la publication de E.R. Andresen et al. (« Measurement and compensation of residual group delay in a multi-core fiber for lensless endoscopy", JOSA B, Vol. 32, No. 6, 1221 - 1228 (2015)).

Il est également possible de compenser le GDD observé sur la FIG. 10A avec un paquet de fibres présentant des coeurs d'indice différents en fonction de la distance au coeur central (indice plus faible sur la périphérie) et/ou en faisant varier la taille des coeurs (les plus petits coeurs présentant des constantes de propagation plus petites et donc des vitesses de groupe plus petites).

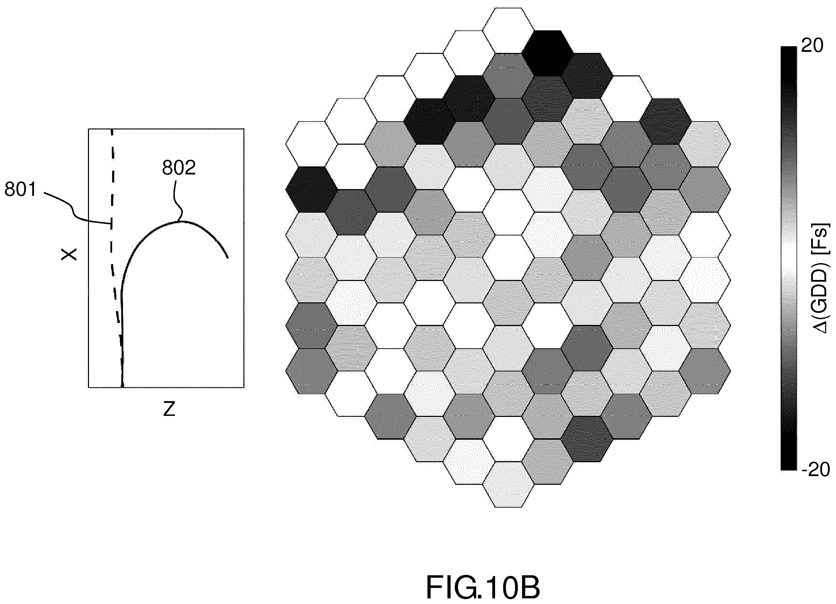

La FIG. 10B illustre la différence de GDD mesuré entre une fibre sans courbure 801 et une fibre courbée 802 avec une courbure de 135° (correspondant à un rayon de courbure de 20 cm environ). On observe que la courbure n'apporte pas de GDD dans le cas d'une MCF torsadée selon la présente description puisque la différence de GDD est de l'ordre de 40fs, c'est-à-dire bien inférieure à la durée des impulsions laser utilisées qui est de l'ordre de 150fs. Ainsi, quand la fibre torsadée est courbée, le GDD mesuré reste constant de telle sorte que la courbure de la MCF n'amène pas de GDD supplémentaire comme c'est le cas dans des MCF non torsadées et courbées, décrites dans « V. Tsvirkun et al., "Bending-induced inter-core group delays in multicorefibers" Optics Express 25, 31863-31875 (2017) ».

Bien que décrits à travers un certain nombre d'exemples de réalisation détaillés, le dispositif de transport et de contrôle de faisceaux lumineux comprend différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent. Notamment, le dispositif de transport et de contrôle de faisceaux lumineux selon la présente description s'applique à l'imagerie endo-microscopique dite « sans lentille » et à toute autre application basée sur l'utilisation d'un paquet de fibres monomodes non couplées pour le transport de faisceaux lumineux et nécessitant le contrôle en phase des faisceaux lumineux en sortie du paquet de fibres optiques monomodes.

## Revendications

1. Dispositif de transport et de contrôle de faisceaux lumineux comprenant :
- un guide de lumière (40) comprenant un paquet (50) de fibres optiques monomodes (Fᵢ) non couplées, chaque fibre optique monomode (Fᵢ) étant destinée à recevoir un faisceau lumineux élémentaire (B₁ᵢ) à une extrémité proximale et à émettre un faisceau lumineux (B₂ᵢ) à une extrémité distale, ledit paquet de fibres optiques monomodes comprenant, en opération, un rayon de courbure minimal correspondant à une courbure maximale du paquet de fibres ;
- un dispositif optique de contrôle de la phase agencé du côté de l'extrémité proximale du guide de lumière (40) comprenant :
- au moins un premier modulateur spatial de lumière (30) adapté pour l'application d'un déphasage sur chacun des faisceaux élémentaires (B₁ᵢ) ;
- une unité de contrôle (60) du premier modulateur spatial de lumière configurée pour appliquer un déphasage sur chacun des faisceaux élémentaires (B₁ᵢ) pour former à l'extrémité distale du guide de lumière un faisceau d'illumination avec une fonction de phase prédéterminée,
et dans lequel ledit paquet (50) de fibres optiques monomodes est torsadé, et comprend une période de torsade (P) déterminée pour conserver ladite fonction de phase à l'extrémité distale du guide de lumière lorsque le paquet de fibres optiques monomodes subit une courbure inférieure à ladite courbure maximale.

2. Dispositif de transport et de contrôle de faisceaux lumineux selon la revendication 1, dans lequel la période de torsade est comprise entre 1 mm et 30 mm, avantageusement entre 2,5 mm et 10 mm.

3. Dispositif de transport et de contrôle de faisceaux lumineux selon l'une quelconque des revendications précédentes, dans lequel la longueur (L) du paquet de fibres optiques monomodes est égale à *k* fois la période de torsade (P) où *k* est un entier.

4. Dispositif de transport et de contrôle de faisceaux lumineux selon l'une quelconque des revendications précédentes, dans lequel l'unité de contrôle (60) du premier modulateur spatial de lumière est configurée en outre pour appliquer une déviation angulaire (θᵢ) sur chacun des faisceaux élémentaires (B₁ᵢ) à l'entrée proximale dudit paquet de fibres optiques monomodes torsadé, ladite déviation angulaire (θᵢ) étant déterminée en fonction de la position de la fibre monomode destinée à recevoir ledit faisceau élémentaire dans ledit paquet de fibres optiques monomodes pour améliorer le couplage dans ladite fibre monomode.

5. Dispositif de transport et de contrôle de faisceaux lumineux selon l'une quelconque des revendications précédentes, dans lequel le guide de lumière comprend à l'extrémité distale et/ou à l'extrémité proximale dudit paquet (50) de fibres optiques monomodes torsadé un tronçon (51, 52) de torsade variable, dans lequel la période de torsade tend vers l'infini du côté de ladite extrémité distale et/ou proximale.

6. Dispositif de transport et de contrôle de faisceaux lumineux selon la revendication 5, dans lequel ledit tronçon de torsade variable présente à ladite extrémité distale et/ou proximale un tronçon non torsadé présentant une longueur inférieure à 1 cm.

7. Dispositif de transport et de contrôle de faisceaux lumineux selon l'une quelconque des revendications précédentes, adapté au transport et au contrôle de faisceaux lumineux comprenant des impulsions optiques, ledit dispositif comprenant en outre un dispositif de contrôle des retards de vitesse de groupe des impulsions lumineuses configuré pour supprimer les retards statiques de vitesse de groupe entre les fibres monomodes dudit paquet de fibres optiques monomodes torsadé.

8. Dispositif de transport et de contrôle de faisceaux lumineux selon l'une quelconque des revendications précédentes, dans lequel au moins une partie desdites fibres optiques monomodes sont dopées.

9. Système d'imagerie endo-microscopique comprenant :
- une source lumineuse (10) pour l'émission de faisceaux lumineux ;
- un dispositif selon l'une quelconque des revendications précédentes pour le transport et le contrôle des faisceaux lumineux émis par ladite source pour la formation d'un faisceau d'illumination d'un objet avec une fonction de phase déterminée; et
- une voie de détection (20, 21) destinée à la détection de la lumière renvoyée par l'objet et transmise à travers ledit au moins un premier guide de lumière (40), de son extrémité distale à son extrémité proximale.

10. Procédé de transport et de contrôle de faisceaux lumineux comprenant :
- la réception de faisceaux lumineux élémentaires (B₁ᵢ) à une extrémité proximale d'un paquet de N fibres optiques monomodes (Fᵢ) d'un guide de lumière, dans lequel :
o chaque fibre optique monomode (Fᵢ) est destinée à recevoir un faisceau lumineux élémentaire (B₁ᵢ) et à émettre un faisceau lumineux (B₂ᵢ) à une extrémité distale ;
o ledit paquet de fibres optiques monomodes comprend, en opération, un rayon de courbure minimal correspondant à une courbure maximale du paquet de fibres; et
∘ledit paquet de fibres optiques monomodes est torsadé, et comprend une période de torsade ;
- l'application au moyen d'au moins un premier modulateur spatial de lumière agencé du côté de l'extrémité proximale dudit paquet de fibres optiques monomodes d'un déphasage sur chacun des faisceaux élémentaires, afin de former à l'extrémité distale du guide de lumière un faisceau d'illumination (B₃) avec une fonction de phase déterminée, ladite période de torsade étant déterminée pour conserver ladite fonction de phase à l'extrémité distale du guide de lumière lorsque le paquet de fibres optiques monomodes subit une courbure inférieure à ladite courbure maximale.

11. Procédé de transport et de contrôle de faisceaux lumineux selon la revendication 10, comprenant en outre l'application d'une déviation angulaire (θᵢ) sur chacun des faisceaux lumineux élémentaires (B₁ᵢ) à l'entrée proximale dudit paquet de fibres optiques monomodes torsadé, ladite déviation angulaire (θᵢ) étant déterminée en fonction de la position de la fibre monomode (Fᵢ) destinée à recevoir ledit faisceau élémentaire dans ledit paquet de fibres optiques monomodes pour améliorer le couplage dans ladite fibre monomode.

12. Procédé de transport et de contrôle de faisceaux lumineux selon l'une quelconque des revendications 10 ou 11, adapté au transport et au contrôle de faisceaux lumineux comprenant des impulsions optiques, ledit procédé comprenant en outre la suppression des retards statiques de vitesse de groupe entre les fibres monomodes dudit paquet de fibres optiques monomodes torsadé au moyen d'un dispositif de contrôle des retards de vitesse de groupe des impulsions lumineuses.

13. Procédé de transport et de contrôle de faisceaux lumineux selon l'une quelconque des revendications 10 à 12, comprenant en outre une calibration préalable permettant de déterminer le déphasage à appliquer sur chacun des faisceaux élémentaires (B₁ᵢ) en fonction de la fonction de phase recherchée pour le faisceau d'illumination (B₃).

14. Procédé d'imagerie endo-microscopique sans lentille du coté distal comprenant :
- l'émission de faisceaux lumineux ;
- le transport et le contrôle des faisceaux lumineux au moyen d'un procédé tel que décrit selon l'une quelconque des revendications 10 à 13 pour l'illumination d'un objet par ledit faisceau d'illumination (B₃);
- la détection de la lumière renvoyée par l'objet et transmise à travers le guide de lumière (50), de son extrémité distale à son extrémité proximale.

## Patentansprüche

1. Vorrichtung zum Transport und zur Steuerung von Lichtstrahlen, aufweisend:
- einen Lichtleiter (40), der ein Bündel (50) ungekoppelter Singlemode-Lichtleitfasern (Fᵢ) aufweist, wobei jede Singlemode-Lichtleitfaser (Fᵢ) dazu bestimmt ist, an einem proximalen Ende einen elementaren Lichtstrahl (B₁ᵢ) zu empfangen und an einem distalen Ende einen Lichtstrahl (B₂ᵢ) auszusenden, wobei das Bündel von Singlemode-Lichtleitfasern im Betrieb einen minimalen Krümmungsradius aufweist, der einer maximalen Krümmung des Faserbündels entspricht;
- eine optische Vorrichtung zur Phasensteuerung, die auf der Seite des proximalen Endes des Lichtleiters (40) angeordnet ist, aufweisend:
- mindestens einen ersten räumlichen Lichtmodulator (30), der zum Anlegen einer Phasenverschiebung an jeden der Elementarstrahlen (B₁ᵢ) geeignet ist;
- eine Steuereinheit (60) des ersten räumlichen Lichtmodulators, die so konfiguriert ist, dass sie eine Phasenverschiebung auf jeden der Elementarstrahlen (B₁ᵢ) anwendet, um am distalen Ende des Lichtleiters einen Beleuchtungsstrahl mit einer vorbestimmten Phasenfunktion zu bilden,
und wobei das Paket (50) aus Singlemode-Lichtleitfaser verdrillt ist und eine Verdrillungsperiode (P) aufweist, die so bestimmt ist, dass die Phasenfunktion am distalen Ende des Lichtleiters beibehalten wird, wenn das Bündel aus Singlemode-Lichtleitfasern eine Krümmung erfährt, die geringer ist als die maximale Krümmung.

2. Vorrichtung zum Transport und zur Steuerung von Lichtstrahlen nach Anspruch 1, wobei die Verdrillungsperiode zwischen 1 mm und 30 mm, vorteilhafterweise zwischen 2,5 mm und 10 mm, liegt.

3. Vorrichtung zum Transport und zur Steuerung von Lichtstrahlen nach einem der vorhergehenden Ansprüche, wobei die Länge (L) des Bündels aus Singlemode-Lichtleitfasern gleich dem k-fachen der Verdrillungsperiode (P) ist, wobei k eine ganze Zahl ist.

4. Vorrichtung zum Transport und zur Steuerung von Lichtstrahlen nach einem der vorhergehenden Ansprüche, wobei die Steuereinheit (60) des ersten räumlichen Lichtmodulators zusätzlich so konfiguriert ist, dass sie eine Winkelablenkung (θᵢ) auf jeden der Elementarstrahlen (B₁ᵢ) am proximalen Eingang des verdrillten Bündels aus Singlemode-Lichtleitfasern anwendet, wobei die Winkelablenkung (θᵢ) in Abhängigkeit von der Position der Singlemode-Lichtleitfaser bestimmt wird, die dazu bestimmt ist, den Elementarstrahl in dem Bündel aus Singlemode-Lichtleitfasern zu empfangen, um die Kopplung in der Singlemode-Lichtleitfaser zu verbessern.

5. Vorrichtung zum Transport und zur Steuerung von Lichtstrahlen nach einem der vorhergehenden Ansprüche, wobei der Lichtleiter am distalen Ende und/oder am proximalen Ende des verdrillten Bündels aus Singlemode-Lichtleitfasern (50) einen Abschnitt (51, 52) mit variabler Verdrillung aufweist, wobei die Verdrillungsperiode auf der Seite des distalen und/oder proximalen Endes gegen unendlich tendiert.

6. Vorrichtung zum Transport und zur Steuerung von Lichtstrahlen nach Anspruch 5, wobei der Abschnitt mit variabler Verdrillung an dem distalen und/oder proximalen Ende einen unverdrillten Abschnitt mit einer Länge von weniger als 1 cm aufweist.

7. Vorrichtung zum Transport und zur Steuerung von Lichtstrahlen nach einem der vorhergehenden Ansprüche, geeignet zum Transport und zur Steuerung von Lichtstrahlen, die optische Impulse aufweisen, wobei die Vorrichtung weiterhin eine Vorrichtung zur Steuerung der Gruppengeschwindigkeitsverzögerungen der Lichtimpulse aufweist, die so konfiguriert ist, dass sie statische Gruppengeschwindigkeitsverzögerungen zwischen den Singlemode-Lichtleitfasern des verdrillten Bündels aus Singlemode-Lichtfasern unterdrückt.

8. Vorrichtung zum Transport und zur Steuerung von Lichtstrahlen nach einem der vorhergehenden Ansprüche, wobei mindestens ein Teil der Singlemode-Lichtleitfaser dotiert ist.

9. System zur endomikroskopischen Bildgebung, aufweisend:
- einer Lichtquelle (10) zum Aussenden von Lichtstrahlen;
- eine Vorrichtung nach einem der vorhergehenden Ansprüche zum Transport und zur Steuerung der von der Quelle emittierten Lichtstrahlen, um einen Beleuchtungsstrahl eines Objekts mit einer bestimmten Phasenfunktion zu bilden; und
- einen Erfassungspfad (20, 21) zum Erfassen von Licht, das von dem Objekt zurückgeworfen und durch den mindestens einen ersten Lichtleiter (40) von seinem distalen Ende zu seinem proximalen Ende übertragen wird.

10. Verfahren zum Transport und zur Steuerung von Lichtstrahlen, aufweisend:
- Empfangen von elementaren Lichtstrahlen (B₁ᵢ) an einem proximalen Ende eines Bündels von N Singlemode-Lichtleitfasern (Fᵢ) eines Lichtleiters, wobei:
- jede Singlemode-Lichtleitfaser (Fᵢ) dazu bestimmt ist, einen elementaren Lichtstrahl (B₁ᵢ) zu empfangen und einen Lichtstrahl (B₂ᵢ) an einem distalen Ende auszusenden;
- das Bündel aus Singlemode-Lichtleitfasern im Betrieb einen minimalen Krümmungsradius aufweist, der einer maximalen Krümmung des Faserbündels entspricht; und
- das Bündel aus Singlemode-Lichtleitfasern verdrillt ist und eine Verdrillungsperiode aufweist;
- Anlegen einer Phasenverschiebung an jeden der Elementarstrahlen mittels mindestens eines ersten räumlichen Lichtmodulators, der auf der Seite des proximalen Endes des Bündels aus Singlemode-Lichtleitfasern angeordnet ist, um am distalen Ende des Lichtleiters einen Beleuchtungsstrahl (B₃) mit einer bestimmten Phasenfunktion zu bilden, wobei die Verdrillungsperiode bestimmt wird, um die Phasenfunktion am distalen Ende des Lichtleiters beizubehalten, wenn das Bündel aus Singlemode-Lichtleitfasern eine Krümmung erfährt, die kleiner als die maximale Krümmung ist.

11. Verfahren zum Transport und zur Steuerung von Lichtstrahlen nach Anspruch 10, das außerdem das Anlegen einer Winkelablenkung (θᵢ) an jeden der Elementarlichtstrahlen (B₁ᵢ) am proximalen Eingang des verdrillten Bündels aus Singlemode-Lichtleitfasern aufweist, wobei die Winkelablenkung (θᵢ) in Abhängigkeit von der Position der Singlemode-Lichtleitfaser (Fᵢ) bestimmt wird, die den Elementarstrahl in dem Bündel aus Singlemode-Lichtleitfasern empfangen soll, um die Kopplung in der Singlemode-Lichtleitfaser zu verbessern.

12. Verfahren zum Transportieren und Steuern von Lichtstrahlen nach einem der Ansprüche 10 oder 11, geeignet zum Transportieren und Steuern von Lichtstrahlen, die optische Impulse aufweisen, wobei das Verfahren ferner das Unterdrücken von statischen Gruppengeschwindigkeitsverzögerungen zwischen den Singlemode-Lichtleitfasern des verdrillten Bündels aus Singlemode-Lichtleitfasern mittels einer Vorrichtung zum Steuern der Gruppengeschwindigkeitsverzögerungen der Lichtimpulse aufweist.

13. Verfahren zum Transport und zur Steuerung von Lichtstrahlen nach einem der Ansprüche 10 bis 12, das außerdem eine vorherige Kalibrierung aufweist, die es ermöglicht, die auf jeden der Elementarstrahlen (B₁ᵢ) anzuwendende Phasenverschiebung in Abhängigkeit von der für den Beleuchtungsstrahl (B₃) angestrebten Phasenfunktion zu bestimmen.

14. Verfahren zur linsenlosen endomikroskopischen Bildgebung der distalen Seite, aufweisend:
- das Aussenden von Lichtstrahlen;
- Transport und Steuerung der Lichtstrahlen mittels eines Verfahrens, wie es nach einem der Ansprüche 10 bis 13 beschrieben ist, zur Beleuchtung eines Objekts durch den Beleuchtungsstrahl (B₃);
- Erfassen von Licht, das von dem Objekt zurückgeworfen und durch den Lichtleiter (50) von seinem distalen Ende zu seinem proximalen Ende geleitet wird.

## Claims

1. A device for transporting and controlling light beams, comprising:
a light guide (40) comprising a bundle (50) of uncoupled single-mode optical fibers (Fᵢ), each single-mode optical fiber (Fᵢ) being intended to receive an elementary light beam (B₁ᵢ) at a proximal end and to emit a light beam (B₂ᵢ) at a distal end, said bundle of single-mode optical fibers comprising, in operation, a minimum radius of curvature corresponding to a maximum curvature of the bundle of fibers;
an optical device for phase controlling, said device being arranged on the side of the proximal end of the light guide (40) and comprising:
at least a first spatial light modulator (30) suitable for applying a phase shift to each of the elementary beams (B₁ᵢ);
a control unit (60) for controlling the first spatial light modulator, said control unit being configured to apply a phase shift to each of the elementary beams (B₁ᵢ) so as to form, at the distal end of the light guide, an illumination beam with a predetermined phase function,
and wherein said bundle (50) of single-mode optical fibers is twisted, and comprises a twist period (P) determined to maintain said phase function at the distal end of the light guide when the bundle of single-mode optical fibers undergoes a curvature lower than said maximum curvature.

2. The device for transporting and controlling light beams as claimed in claim 1, wherein the twist period is comprised between 1 mm and 30 mm, and advantageously between 2.5 mm and 10 mm.

3. The device for transporting and controlling light beams as claimed in any one of the preceding claims, wherein the length (L) of the bundle of single-mode optical fibers is equal to *k* times the twist period (P) where *k* is an integer.

4. The device for transporting and controlling light beams as claimed in any one of the preceding claims, wherein the control unit (60) for controlling the first spatial light modulator is furthermore configured to apply an angular deviation (θᵢ) to each of the elementary beams (B₁ᵢ) at the proximal entrance of said twisted bundle of single-mode optical fibers, said angular deviation (θᵢ) being determined, depending on the position of the single-mode fiber intended to receive said elementary beam in said bundle of single-mode optical fibers, so as to improve coupling to said single-mode fiber.

5. The device for transporting and controlling light beams as claimed in any one of the preceding claims, wherein the light guide comprises at the distal end and/or at the proximal end of said twisted bundle (50) of single-mode optical fibers a section (51, 52) of variable twist, wherein the twist period tends to infinity on the side of said distal and/or proximal end.

6. The device for transporting and controlling light beams as claimed in claim 5, wherein said section of variable twist has, at said distal and/or proximal end, an untwisted section having a length smaller than 1 cm.

7. The device for transporting and controlling light beams as claimed in any one of the preceding claims, said device being suitable for transporting and controlling light beams comprising optical pulses, said device furthermore comprising a device for controlling the group velocity delays of the light pulses, the latter device being configured to suppress static group velocity delays between the single-mode fibers of said twisted bundle of single-mode optical fibers.

8. The device for transporting and controlling light beams as claimed in any one of the preceding claims, wherein at least some of said single-mode optical fibers are doped.

9. An endomicroscopic imaging system comprising:
a light source (10) for emitting light beams;
a device as claimed in any one of the preceding claims, for transporting and controlling the light beams emitted by said source so as to form a beam for illuminating an object with a determined phase function; and
a detection channel (20, 21) intended for the detection of the light returned by the object and transmitted through said at least one first light guide (40), from its distal end to its proximal end.

10. A method for transporting and controlling light beams, comprising:
receiving elementary light beams (B₁ᵢ) at a proximal end of a bundle of N single-mode optical fibers (Fᵢ) of a light guide, wherein:
each single-mode optical fiber (Fᵢ) is intended to receive an elementary light beam (B₁ᵢ) and to emit a light beam (B₂ᵢ) at a distal end;
said bundle of single-mode optical fibers comprises, in operation, a minimum radius of curvature corresponding to a maximum curvature of the bundle of fibers; and
said bundle of single-mode optical fibers is twisted, and comprises a twist period;
applying, by means of at least a first spatial light modulator arranged on the side of the proximal end of said bundle of single-mode optical fibers, a phase shift to each of the elementary beams, in order to form, at the distal end of the light guide, an illumination beam (B₃) with a determined phase function, said twist period being determined to maintain said phase function at the distal end of the light guide when the bundle of single-mode optical fibers undergoes a curvature lower than said maximum curvature.

11. The method for transporting and controlling light beams as claimed in claim 10, furthermore comprising applying an angular deviation (θᵢ) to each of the elementary light beams (B₁ᵢ) at the proximal entrance of said twisted bundle of single-mode optical fibers, said angular deviation (θᵢ) being determined, depending on the position of the single-mode fiber (Fᵢ) intended to receive said elementary beam in said bundle of single-mode optical fibers, so as to improve coupling to said single-mode fiber.

12. The method for transporting and controlling light beams as claimed in either one of claims 10 and 11, said method being suitable for transporting and controlling light beams comprising optical pulses, said method furthermore comprising suppressing static group velocity delays between the single-mode fibers of said twisted bundle of single-mode optical fibers by means of a device for controlling the group velocity delays of the light pulses.

13. The method for transporting and controlling light beams as claimed in any one of claims 10 to 12, furthermore comprising a prior calibration allowing the phase shift to be applied to each of the elementary beams (B₁ᵢ) depending on the phase function sought for the illumination beam (B₃) to be determined.

14. An endomicroscopic imaging method employing no lens distal side, comprising:
emitting light beams;
transporting and controlling the light beams by means of a method as claimed in any one of claims 10 to 13 so as to illuminate an object with said illumination beam (B₃);
detecting the light returned by the object and transmitted through the light guide (50), from its distal end to its proximal end.
